# EUROPEAN PATENT APPLICATION

(11) **EP 4 803 097 A1**
(43) Date of publication of application: **09.09.2026**
(21) Application number: 24884552.1
(22) Date of filing: 24.10.2024
(51) Int. Cl.: A61K 39/00, C07K 19/00, C12N 15/62, A61P 31/06

(54) **NANOPARTICLE VACCINE FOR PREVENTING MYCOBACTERIUM TUBERCULOSIS INFECTION AND PREPARATION METHOD THEREFOR**

(30) Priority: 03.11.2023 CN 202311457722
(71) Applicant: Yantai Patronus Biotech Co., Ltd., Yantai, Shandong 264670 (CN); Guangzhou Patronus Biotech Co., Ltd., Guangzhou, Guangdong 510000 (CN)
(72) Inventor: DING, Yanbin, Guangzhou, Guangdong 510000 (CN); LI, Yuanyuan, Guangzhou, Guangdong 510000 (CN); JIN, Jing, Guangzhou, Guangdong 510000 (CN)
(74) Representative: karo IP
(86) International application number: PCT/CN2024/126948
(87) International publication number: WO 2025/092548

(57) **Abstract**

Disclosed is a Mycobacterium tuberculosis vaccine. Specifically, the vaccine comprises an immune composition comprising an antigen component and a particulate protein component. The particulate protein component comprises nanoparticle protein. The antigen component and the particulate protein component are covalently bound by means of a binding peptide 1 and a binding peptide 2 to form an immunogenic complex. The vaccine has excellent cell immunogenicity and antibody immunogenicity. The present invention also relates to a preparation method for the mycobacterium tuberculosis vaccine.

## Description

### TECHNICAL FIELD

The present disclosure relates to the field of biopharmaceutical technology, and in particular to an immune composition product for preventing *Mycobacterium tuberculosis* infection and a method for preparing same.

### BACKGROUND

Tuberculosis is a chronic infectious disease caused by *M. tuberculosis* and other mycobacterial infections. The major transmission route of the disease is droplet transmission. The disease may affect people in any age group, and those with hypoimmunity are more susceptible to the infection. Those infected may not have typical symptoms for a long time, but develop symptoms such as acute lung inflammation, dry cough, and fever, which may generally, if not treated in time, directly lead to serious complications and deaths.

At present, tuberculosis is a major disease in developing countries and is widely distributed worldwide with a growing trend. There were 10.6 million new tuberculosis cases worldwide in 2021, and 1.6 million people died from tuberculosis, equivalent to about 4300 deaths every day. Tuberculosis mainly affects people in mid- and low-income countries, and those poor people with poor living and working conditions and malnutrition are often at the highest risk of tuberculosis. Globally, as many as one fourth of the population is infected with latent tuberculosis. The tuberculosis bacterium infection exhibits no symptoms, but may progress to active tuberculosis. At present, the use of drugs such as isoniazid, rifampicin, and pyrazinamide as the antibiotic treatment of tuberculosis patients controls tuberculosis, but such treatment cannot effectively prevent the spread of the disease. After infection, individuals do not develop symptoms within a period of time, but may have certain infectivity. In addition, the long-term use of antibiotics poses a great challenge to the compliance of patients. Some patients may not even complete the effective treatment. Meanwhile, drug resistance may occur in long-term treatments with antibiotics.

Effective vaccination and accurate early diagnosis are important means for controlling tuberculosis. At present, the Bacillus Calmette-Guerin (BCG) vaccine for the vaccination of infants is prepared from the non-toxic *Mycobacterium bovis*. The widespread use of the BCG vaccine provides a great help in global tuberculosis prevention and treatment. The BCG vaccine can protect infants from severe systemic tuberculosis, but has very limited protective effects on tuberculosis in adolescents and adults. Therefore, there is an urgent need to develop a novel tuberculosis vaccine that can completely replace the BCG vaccine or the BCG booster vaccine. Tuberculosis vaccines in the form of a recombinant subunit have demonstrated potency in humans and gradually become a hot spot for the development of novel tuberculosis vaccines. For example, in a novel tuberculosis vaccine candidate M72/AS01E developed by GSK, M72 is a recombinant fusion protein containing two antigens of *M. tuberculosis* (MTB32A and MTB39A). However, the phase II clinical data suggest a protection rate of only 49.7%, barely meeting the TB vaccine requirement of WHO. Although the vaccine has certain immune efficacy, the M72 fusion protein is prone to degradation in the expression process and will produce inclusion bodies, and the particle size of the fusion protein is greatly affected by salt ions and pH, resulting in a complex production process and cost-inefficiency. Therefore, it is of great significance to develop a novel TB vaccine with a higher protection rate and a simple production process for human vaccination.

At present, as a new generation of vaccine design, nanoparticle vaccines can not only induce potent neutralizing antibodies, but also improve the cellular immune response level. Among these, human HPV vaccines, hepatitis B vaccines, veterinary PCV2 vaccines, and the like are representative. There are many natural proteins (ferritin, lumazine synthase, Mi3, AP205, etc.) in nature that can self-assemble into nanoparticles and induce a strong immune response after loading antigens on the surface, and have been widely explored and applied at present. The body's resistance to *M. tuberculosis* infection mainly depends on the cellular immune mechanism. Vaccine immunization can produce effective Th1 CD4+ T and CD8+ T levels and long-lasting cellular immune memory, which is the key point of the development of novel tuberculosis vaccines. Therefore, self-assembled nanoparticles are very ideal carriers and are feasible in the exploration of tuberculosis vaccines.

### SUMMARY

The present disclosure provides an *M. tuberculosis* vaccine and a method for preparing same. The vaccine is a nanoparticle vaccine.

The nanoparticle vaccine is a vaccine based on a nanoparticle protein, which is mainly used for antigen display. The present disclosure provides an immunogenic complex, comprising a protein formed by a covalent binding reaction between an antigenic component and a particle protein component.

The present disclosure provides an immune composition, comprising the immunogenic complex disclosed herein and a pharmaceutically acceptable carrier. The immune composition may be in the form of a lyophilized dosage form, an injection dosage form, an oral dosage form, or a spray dosage form.

The present disclosure provides a vaccine, comprising the immune composition disclosed herein and an adjuvant.

The present disclosure provides an immunogenic complex, comprising:
(1) an antigenic component, comprising an *M. tuberculosis* structural protein or an immunogenic fragment thereof; and
(2) a particle protein component, comprising a nanoparticle protein.

The present disclosure provides an immunogenic complex, comprising:
(1) an antigenic component, comprising an *M. tuberculosis* structural protein or an immunogenic fragment thereof, a linker
   peptide 1, and a binding peptide 1; and
(2) a particle protein component, comprising a nanoparticle protein, a linker peptide 2, and a binding peptide 2, wherein the antigenic component and the particle protein component are covalently bound to each other via the binding peptide 1 and the binding peptide 2.

The present disclosure provides an immunogenic complex, comprising:
(1) an antigenic component, consisting of an *M. tuberculosis* structural protein or an immunogenic fragment thereof, a linker
   peptide 1, and a binding peptide 1; and
(2) a particle protein component, consisting of a nanoparticle protein, a linker peptide 2, and a binding peptide 2, wherein the antigenic component and the particle protein component are covalently bound to each other via the binding peptide 1 and the binding peptide 2.

In some embodiments, in any one of the immunogenic complexes provided herein, the antigenic component is formed by fusing the *M. tuberculosis* structural protein, at the C-terminus, with the binding peptide 1 via the linker peptide 1.

In some embodiments, the "immunogenic fragment" refers to a portion of an oligopeptide, a polypeptide, or a protein that is immunogenic and elicits a protective immune response when administered to a subject.

In some embodiments, in any one of the immunogenic complexes provided herein, the particle protein component is formed by fusing the nanoparticle protein, at the N-terminus, with the binding peptide 2 via the linker peptide 2.

In some embodiments, in any one of the immunogenic complexes provided herein, the antigenic component is, from the N-terminus to the C-terminus: the *M. tuberculosis* structural protein or the immunogenic fragment thereof, the linker peptide 1, and the binding peptide 1; the particle protein component is, from the N-terminus to the C-terminus: the binding peptide 2, the linker peptide 2, and the nanoparticle protein; the antigenic component and the particle protein component are covalently bound to each other via the binding peptide 1 and the binding peptide 2, thus forming the immunogenic complex.

In some embodiments, in any one of the immunogenic complexes provided herein, the antigenic component and/or the particle protein component comprise a histidine tag.

The present disclosure provides an immunogenic complex, comprising:
(1) an antigenic component, comprising an *M. tuberculosis* structural protein or an immunogenic fragment thereof, and a linker peptide 1; and
(2) a particle protein component, comprising a nanoparticle protein subunit.

In some embodiments, the *M. tuberculosis* structural protein is linked to one subunit of the nanoparticle protein to form a fusion protein, and the fusion protein then binds to the other subunit of the nanoparticle protein.

In some embodiments, in any one of the immunogenic complexes provided herein, the particle protein component comprises a nanoparticle protein. Preferably, the nanoparticle protein may be a virus-like particle protein formed from a viral structural protein, preferably from bacteriophage capsid protein AP205. The particle protein component and the antigenic component may be covalently bound to form a particle structure.

In some embodiments, in any one of the immunogenic complexes provided herein, the nanoparticle protein used may also be selected from an NPM particle, a ferritin particle, an I53-50 particle, a lumazine synthase (LS) particle, or the like.

In some embodiments, in any one of the immunogenic complexes provided herein, the nanoparticle protein I53-50 particle used consists of two subunits I53-50A and I53-50B.

In some embodiments, in any one of the immunogenic complexes provided herein, the binding peptide 1 comprises the amino acid sequence set forth in SEQ ID NO: 1.

In some embodiments, in any one of the immunogenic complexes provided herein, the binding peptide 2 comprises the amino acid sequence set forth in SEQ ID NO: 24.

In some embodiments, in any one of the immunogenic complexes provided herein, the linker peptide 1 comprises the amino acid sequence of (GSG)ₙ, (GGGGS)ₙ, or (EAAAK)ₙ, wherein n may be an integer greater than 0 and less than or equal to 5. In some embodiments, in any one of the immunogenic complexes provided herein, the linker peptide 1 is preferably GSG GSG (SEQ ID NO: 2).

In some embodiments, in any one of the immunogenic complexes provided herein, the linker peptide 2 comprises the amino acid sequence of (GGS)ₙ, (SGGSGG)ₙ, or (GSGGSGGSG)ₙ, wherein n may be an integer greater than 0 and less than or equal to 10. In some embodiments, in any one of the immunogenic complexes provided herein, the linker peptide 2 is preferably GGSGGSGGSGGS (SEQ ID NO: 25).

Specifically, the structural protein of *M. tuberculosis* of the present disclosure uses antigens Mtb32a or Ag85a or fusion proteins ESAT6-CFP10 or RV2660-TB10.4, such that the protein described above is connected, at the C-terminus, to the binding peptide 1 (designated as "4T") through a specific linker peptide 1 (linker 1), and meanwhile a histidine (e.g., 6His, i.e., HHHHHH) purification tag can be added to the C-terminus of the fusion protein; a coding gene encoding the *M. tuberculosis* structural protein described above is inserted into a prokaryotic cell expression vector (e.g., pET21a) for expression in *Escherichia coli* BL21(DE3) cells to produce a fusion protein formed by the structural protein of *M. tuberculosis* and the binding peptide 1; the antigenic components are subjected to nickel column affinity chromatography, size-exclusion chromatography, and the like to obtain high-purity proteins; the antigenic component is Mtb32a-4T, Ag85a-4T, ESAT6-CFP10-4T, or RV2660-TB10.4-4T.

Preferably, the structural protein of *M. tuberculosis* of the present disclosure uses antigens Mtb32a or Ag85a or fusion proteins ESAT6-CFP10 or RV2660-TB10.4, wherein the sequence of Mtb32a is set forth in SEQ ID NO: 3, the sequence of Ag85a is set forth in SEQ ID NO: 5, the sequence of ESAT6-CFP10 is set forth in SEQ ID NO: 7, and the sequence of RV2660-TB10.4 is set forth in SEQ ID NO: 9.

Preferably, the structural protein of *M. tuberculosis* of the present disclosure uses antigens Mtb32a or Ag85a or fusion proteins ESAT6-CFP10 or RV2660-TB10.4, wherein the sequence of Mtb32a has 40% or higher, 50% or higher, 60% or higher, 70% or higher, 80% or higher, or 90% or higher identity to SEQ ID NO: 3, the sequence of Ag85a has 40% or higher, 50% or higher, 60% or higher, 70% or higher, 80% or higher, or 90% or higher identity to SEQ ID NO: 5, the sequence of ESAT6-CFP10 has 40% or higher, 50% or higher, 60% or higher, 70% or higher, 80% or higher, or 90% or higher identity to SEQ ID NO: 7, and the sequence of RV2660-TB10.4 has 40% or higher, 50% or higher, 60% or higher, 70% or higher, 80% or higher, or 90% or higher identity to SEQ ID NO: 9.

Preferably, in any one of the immunogenic complexes provided herein, the antigenic component comprises *M. tuberculosis* structural protein Mtb32a (SEQ ID NO: 3), Ag85a (SEQ ID NO: 5), ESAT6-CFP10 (SEQ ID NO: 7), or RV2660-TB10.4 (SEQ ID NO: 9), linker peptide 1-GSGGSG (SEQ ID NO: 2), binding peptide 1 (SEQ ID NO: 1), and a histidine tag; more preferably, the sequence of antigenic component Mtb32a-4T is set forth in SEQ ID NO: 4, the sequence of antigenic component Ag85a-4T is set forth in SEQ ID NO: 6, the sequence of antigenic component ESAT6-CFP10-4T is set forth in SEQ ID NO: 8, and the sequence of antigenic component RV2660-TB10.4-4T is set forth in SEQ ID NO: 10.

In some embodiments, in any one of the above immunogenic complexes provided herein, the particle protein component is a fusion protein formed by fusing the nanoparticle protein, at the N-terminus, with the binding peptide 2 via the linker peptide 2; preferably, the nanoparticle protein is NPM, AP205 capsid protein 3 (AP205), or ferritin protein. Specifically, in some optional embodiments, the binding peptide 2 (designated as "4C") is linked to the coding gene of the nanoparticle protein via the linker peptide 2, and the construct is inserted into a prokaryotic expression vector (such as pET-28a(+) or pET-30a(+)) for expression in *E. coli* cells, so as to obtain a fusion protein of the binding peptide 2 and the nanoparticle protein. The fusion protein may be purified by chromatography (e.g., anion exchange chromatography and hydrophobic chromatography) to produce a product. The nanoparticle protein is preferably NPM, AP205, or ferritin; the formed particle protein components are designated as NPM-4C, AP205-4C, and ferritin-4C.

Specifically, in some optional embodiments, any one of the above antigenic components is subjected to a conjugation binding reaction with the particle protein component under suitable reaction conditions, and the coupling is achieved by the formation of a covalent bond between the binding peptide 1 of the antigenic component and the binding peptide 2 of the particle protein component, thereby forming the immunogenic complex. With the use of different nanoparticle proteins, different immunogenic complexes are formed and designated as Mtb32a-NPM, Mtb32a-AP205, or Mtb32a-ferritin; Ag85a-NPM, Ag85a-AP205, or Ag85a-ferritin; ESAT6-CFP10-NPM, ESAT6-CFP10-AP205, or ESAT6-CFP10-ferritin; RV2660-TB10.4-NPM, RV2660-TB10.4-AP205, or RV2660-TB10.4-ferritin.

In some embodiments, the present disclosure provides an immunogenic complex, comprising:
(1) an antigenic component, comprising an *M. tuberculosis* structural protein, a linker peptide 1, and a binding peptide 1; and
(2) a particle protein component, comprising a nanoparticle protein, a linker peptide 2, and a binding peptide 2.

The linker peptide 1 is any linker peptide commonly used in the art, including but not limited to the amino acid sequence of (GSG)ₙ, (GGGGS)ₙ, or (EAAAK)ₙ, wherein n may be an integer greater than 0 and less than or equal to 5, preferably GSGGSG (SEQ ID NO:2); the linker peptide 2 may be any linker peptide commonly used in the art, including but not limited to the amino acid sequence of (GGS)ₙ, (SGG)ₙ, or (GSGGSGGSG)ₙ, wherein n may be an integer greater than 0 and less than or equal to 10, preferably GGSGGSGGSGGS (SEQ ID NO:25). The nanoparticle protein is NPM, AP205, or ferritin.

Preferably, in any one of the immunogenic complexes provided herein, the particle protein component comprises NPM-4C, as set forth in SEQ ID NO: 27, which is a fusion protein obtained by linking the binding peptide 2 set forth in SEQ ID NO: 24 to the nanoparticle protein NPM set forth in SEQ ID NO: 26 via the linker peptide 2 set forth in SEQ ID NO: 25.

In some other embodiments, the present disclosure provides an immunogenic complex, comprising:
(1) an antigenic component, comprising an *M. tuberculosis* structural protein and a linker peptide 1; and
(2) a particle protein component, comprising a nanoparticle protein subunit, wherein preferably, the nanoparticle protein subunit is an I53-50A subunit and/or an I53-50B subunit.

In some embodiments, in any one of the immunogenic complexes provided herein, the nanoparticle protein I53-50 comprises an I53-50A subunit and/or an I53-50B subunit.

Specifically, in any one of the immunogenic complexes provided herein, the *M. tuberculosis* structural protein is linked to one subunit of the nanoparticle protein to form a fusion protein, and the fusion protein then binds to the other subunit of the nanoparticle protein. Preferably, the subunit of the nanoparticle protein is I53-50A or I53-50B. Further, in some optional embodiments, the *M. tuberculosis* structural protein in the antigenic component binds, at the C-terminus, to the nanoparticle protein I53-50A subunit via the linker peptide 1 to form an *M. tuberculosis* structural protein-I53-50A fusion protein, and the fusion protein then binds to the nanoparticle protein I53-50B subunit.

As described above, when I53-50, which comprises two subunits I53-50A and I53-50B, is selected as the nanoparticle protein, the above *M. tuberculosis* structural protein with or without a specific signal peptide is linked to I53-50A via the linker peptide 1, and a histidine (e.g., 6H) purification tag may be added to the C-terminus. The coding gene encoding the above fusion protein is inserted into a eukaryotic cell expression vector (e.g., pcDNA3.4) for expression and purification in CHO cells, and the resulting fusion protein is designated as *M. tuberculosis* structural protein-I53-50A. Also, a histidine (e.g., 6H) purification tag may be added to the C-terminus of I53-50B, and the gene encoding the above protein is inserted into a prokaryotic cell expression vector (e.g., pET-30a(+)) for expression and purification in *E.coli* cells, and the resulting protein is designated as I53-50B. Then, the *M. tuberculosis* structural protein-I53-50A is subjected to a covalent binding reaction with the I53-50B under suitable reaction conditions to form an *M. tuberculosis* nanoparticle, which is designated as *M. tuberculosis* structural protein-I53-50.

Preferably, in any one of the immunogenic complexes provided herein, the *M. tuberculosis* structural protein-I53-50A is comprised.

In some embodiments, the present disclosure provides an immunogenic complex, comprising any one or more of the following (1)-(7):
(1) the amino acid sequence of the *M. tuberculosis* structural protein is set forth in SEQ ID NO: 3, SEQ ID NO: 5, SEQ ID NO: 7, or SEQ ID NO: 9;
(2) the amino acid sequence of the linker peptide 1 is set forth in SEQ ID NO: 2;
(3) the amino acid sequence of the binding peptide 1 is set forth in SEQ ID NO: 1;
(4) the nanoparticle protein is selected from NPM, AP205, or ferritin;
(5) the nanoparticle protein subunit is selected from I53-50A and/or I53-50B;
(6) the linker peptide 2 comprises the amino acid sequence of (GGS)ₙ, (SGGSGG)ₙ, or (GSGGSGGSG)ₙ, wherein n may be an integer greater than 0 and less than or equal to 10; the amino acid sequence of the linker peptide 2 is set forth in SEQ ID NO: 25; and
(7) the amino acid sequence of the binding peptide 2 is set forth in SEQ ID NO: 24.

In some embodiments, the present disclosure provides an immunogenic complex, consisting of an antigenic component and a particle protein component, wherein the amino acid sequence of the antigenic component is set forth in SEQ ID NO: 4, SEQ ID NO: 6, SEQ ID NO: 8, and SEQ ID NO: 10, and the amino acid sequence of the particle protein component is set forth in SEQ ID NO: 27.

Further, the present disclosure also provides a preparation method for any one of the above immunogenic complexes, comprising the following steps:
(1) ligating a coding gene of the antigenic component and a coding gene of the particle protein component into expression vectors to construct recombinant expression plasmids and expression host strains, and expressing and purifying the target proteins; and
(2) co-incubating the antigenic component and the particle protein component obtained in step (1) to obtain the immunogenic complex.

The present disclosure provides a method for preparing an immunogenic complex for preventing or treating an *M. tuberculosis*-related disease:
(1) ligating a coding gene of an *M. tuberculosis* antigenic component and a coding gene of the particle protein component into expression vectors to construct recombinant expression plasmids;
(2) constructing a recombinant strain capable of expressing the *M. tuberculosis* antigenic component and the particle protein component in the host cell;
(3) expressing fusion proteins using the recombinant strain and purifying the fusion proteins; and
(4) co-incubating the antigenic component and the particle protein component described above to perform a conjugation binding reaction, so as to obtain the immunogenic complex.

Preferably, the immunogenic complex obtained in step (4) above is purified to obtain a vaccine drug substance. Preferably, in step (1) of the method for preparing the immunogenic complex for preventing or treating the *M. tuberculosis*-related disease, the plasmid expressing the *M. tuberculosis* antigenic component may be selected from pET21a, and the plasmid expressing the particle protein component may be selected from pET-28a(+) or pET-30a(+).

In step (2) of the method for preparing the immunogenic complex for preventing or treating the *M. tuberculosis-*related disease disclosed herein, the host cell expressing the *M. tuberculosis* antigen is *E. coli*, and the host cell expressing the particle protein component vector is *E. coli*.

The present disclosure provides an immunogenic complex for preventing or treating an *M. tuberculosis-*related disease, wherein an antigenic component of the immunogenic complex comprises the fusion protein formed by the *M. tuberculosis* structural protein - binding peptide 1 described above.

In the immunogenic complex for preventing or treating the *M. tuberculosis*-related disease disclosed herein, high-purity Mtb32a, Ag85a, ESAT6-CFP10, and RV2660-TB10.4 antigens obtained by size-exclusion chromatographic purification and NPM-4C are mixed in a BCA protein concentration ratio of 6:1, a 50% sucrose stock solution is added at a final sucrose concentration of about 25%, and a 1 M Tris-HCl stock solution is added at 10% of the total reaction volume to stabilize the pH. The binding reaction was carried out at 22 °C for 48 h. All endotoxin measurements are below 100 EU/mL, meeting the requirements for mass production.

The present disclosure also provides an immune composition, comprising any one of the above immunogenic complexes and a pharmaceutically acceptable carrier. Preferably, the pharmaceutically acceptable carrier comprises a stabilizer, an excipient, a surfactant, a buffering agent, and a pH regulator. The stabilizer is sucrose and/or arginine, the excipient is mannitol, the surfactant is Tween 80, the buffering agent is disodium hydrogen phosphate dihydrate and/or sodium dihydrogen phosphate dihydrate, and the pH regulator is hydrochloric acid. In some embodiments, the immune composition disclosed herein comprises the immunogenic complex in an amount of 0.25-100 µg/dose, preferably 0.5-50 µg/dose, more preferably 0.5 µg/dose, 1 µg/dose, 2 µg/dose, 3 µg/dose, 4 µg/dose, 5 µg/dose, 10 µg/dose, 15 µg/dose, 20 µg/dose, 25 µg/dose, 30 µg/dose, 35 µg/dose, 40 µg/dose, 45 µg/dose, or 50 µg/dose. The dose for mouse study is 1/10 of the human dose.

In some embodiments, the immune composition provided herein is an injection or a lyophilized formulation, preferably a lyophilized formulation.

In some embodiments, the immune composition provided herein is a lyophilized formulation, comprising an *M. tuberculosis* structural protein-NPM immunogenic complex, a stabilizer, an excipient, a surfactant, a buffering agent, and a pH regulator; preferably, the stabilizer is sucrose and/or arginine, the excipient is mannitol, the surfactant is Tween 80, the buffering agent is disodium hydrogen phosphate dihydrate and/or sodium dihydrogen phosphate dihydrate, and the pH regulator is hydrochloric acid.

In some embodiments, the immune composition provided herein is a lyophilized formulation, comprising an *M. tuberculosis* structural protein-NPM immunogenic complex, sucrose, arginine, mannitol, Tween 80, disodium hydrogen phosphate dihydrate, sodium dihydrogen phosphate dihydrate, and hydrochloric acid.

In some embodiments, the immune composition provided herein is an injection, comprising an *M. tuberculosis* structural protein-NPM immunogenic complex, a stabilizer, a surfactant, a buffering agent, and a pH regulator; preferably, the stabilizer is sucrose, the surfactant is Tween 80, the buffering agent is disodium hydrogen phosphate dihydrate and/or sodium dihydrogen phosphate dihydrate, and the pH regulator is hydrochloric acid. In some embodiments, the immune composition provided herein is an injection, comprising an *M. tuberculosis* structural protein-NPM immunogenic complex, sucrose, Tween 80, disodium hydrogen phosphate dihydrate, sodium dihydrogen phosphate dihydrate, and hydrochloric acid.

The present disclosure further provides an *M. tuberculosis* vaccine, comprising any one of the above immune compositions and an adjuvant. The adjuvant is selected from at least one of an aluminum salt adjuvant, Freund's complete adjuvant, a propolis adjuvant, an oil-in-water adjuvant, a cytokine, CpGDNA, a genetically engineered toxoid, an immune-stimulating complex, or a liposome.

According to the *M. tuberculosis* vaccine disclosed herein, the oil-in-water adjuvant is a squalene-containing squalene-based adjuvant.

According to the *M. tuberculosis* vaccine disclosed herein, the vaccine comprises, per unit dose for human use, 5-50 µg, preferably 5 µg, 25 µg, or 50 µg of the immunogenic complex.

The squalene-based adjuvant disclosed herein comprises: 0.5%-5% (w/w) of squalene, 0.05%-1% of Span 85, 0.05%-1% of Tween 80, and 10 mM of citrate buffer.

The squalene-based adjuvant disclosed herein preferably comprises: 2%-4.5% (w/w) of squalene, 0.2%-0.5% of Span 85, 0.2%-0.5% of Tween 80, and 10 mM of citrate buffer. The amount of the squalene used is more preferably 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, or 4.4% (w/w), the amount of Span 85 is more preferably 0.3%-0.4% (w/w), and the amount of Tween 80 is more preferably 0.3%-0.4% (w/w).

As described above, the amount of the immunogenic complex, *M. tuberculosis* structural protein-NPM, and the adjuvant used in humans and mice is different, and the correspondence is as follows: when used in humans, the amount of *M. tuberculosis* structural protein-NPM and that of the adjuvant are both 10 times the amounts used in mice, e.g., 5 µg/dose of *M. tuberculosis* structural protein-NPM for mice and 50 µg/dose for humans, 50 µL/dose of adjuvant for mice and 500 µL/dose (0.5 mL/dose) of adjuvant for humans, 25 µL/dose of adjuvant for mice and 250 µg/dose (0.25 mL/dose) of adjuvant for humans, and so on.

The present disclosure further provides a kit, comprising the *M. tuberculosis* vaccine disclosed herein and a device and a container for vaccination with the vaccine.

The present disclosure provides an *M. tuberculosis* vaccine, comprising an *M. tuberculosis* structural protein-NPM immune composition (i.e., an immune combination comprising the *M. tuberculosis* structural protein-NPM, which can be prepared into a lyophilized formulation or an injection formulation) and an adjuvant (which is a liquid). The *M. tuberculosis* structural protein-NPM immune composition and the adjuvant are packaged in separate vials.

The present disclosure provides use of the *M. tuberculosis* nanoparticle immunogenic complex, the immune composition, or the vaccine in preparing a medicament for preventing or treating tuberculosis.

All reagents used in the present disclosure are commercially available.

Compared with the prior art, the present disclosure has the following beneficial effects:
1. In the present disclosure, *M. tuberculosis* structural proteins MTB32A-4T, Ag85a-4T, ESAT6-CFP10-4T, and RV2660-TB10.4-4T, are expressed in a fusion expression form using an *E. coli* expression system, and soluble expression of these fusion proteins can be achieved. Also, NPM-4C nanoparticles are prepared by using the *E. coli* expression system. The immunogenic complexes of the present disclosure have a significant prophylactic effect against *M. tuberculosis* (Mtb) infection. Nanoparticles Mtb32a-NPM, Ag85a-NPM, ESAT6-CFP10-NPM, and RV2660-TB10.4-NPM prepared from Mtb structural proteins Mtb32a, Ag85a, ESAT6-CFP10, and RV2660-TB10.4 have the advantages of uniform particle size, uniform distribution, no aggregation, stable product performance, qualified endotoxin, and suitability for non-clinical development and antibody immunogenicity test as a tuberculosis vaccine.
2. Fusion proteins Mtb32a-4T, Ag85a-4T, ESAT6-CFP10-4T, and RV2660-TB10.4-4T were covalently coupled to NPM-4C nanoparticle for the first time to prepare nanoparticle antigens Mtb32a-NPM, Ag85a-NPM, ESAT6-CFP10-NPM, and RV2660-TB10.4-NPM, which a feature stable production process, cost-efficiency, and great industrial potential.
3. The results of animal immunization study show that mice immunized with Mtb32a-NPM, Ag85a-NPM, ESAT6-CFP10-NPM, and RV2660-TB10.4-NPM can produce high levels of specific IgG1 and IgG2a antibodies, indicating that Mtb32a-NPM, Ag85a-NPM, ESAT6-CFP10-NPM, and RV2660-TB10.4-NPM nanoparticles have good immunogenicity and can well induce cellular immunity and humoral immunity.
4. The mixture of Mtb32a-4T, Ag85a-4T, ESAT6-CFP10-4T, and RV2660-TB10.4-4T fusion proteins can significantly reduce *M. tuberculosis* infection, and the mixture of Mtb32a-NPM, Ag85a-NPM, ESAT6-CFP10-NPM, and RV2660-TB10.4-NPM can significantly reduce the load of *M. tuberculosis* in the spleen and lungs of infected mice, which has important development value.
5. The method for preparing the nanoparticle-based tuberculosis vaccine provided herein features cost-efficiency and suitability for mass production. In the present disclosure, the particle protein component is prepared by *E. coli* fermentation and chromatographic purification, and the antigenic components Mtb32a-4T, Ag85a-4T, ESAT6-CFP10-4T, and RV2660-TB10.4-4T can be prepared by cell reactor culture and chromatographic purification, which are both suitable for industrial mass production and possess the advantages of high expression level, stable process and yield, simple operation, and the like. The amount of the recombinant particle protein component in one batch can correspond to multiple batches of antigenic components Mtb32a-4T, Ag85a-4T, ESAT6-CFP10-4T, and RV2660-TB10.4-4T for combination, such that the production efficiency is improved, and the cost of mass production can be saved.
6. The method for preparing the recombinant particle protein component provided herein features ease to operate and reduced amount of organic solvents used in subsequent chromatographic purification, and thus reduced cost in industrial mass production and suitability for industrial production. The protein product prepared by the method for preparing the recombinant particle protein component provided herein, the side effects caused by residues such as impurities, host proteins, organic solvents, exogenous DNAs, antibiotics, bacterial endotoxins, and other substances in the particles are effectively reduced, thereby improving the safety.

### BRIEF DESCRIPTION OF THE DRAWINGS

FIG. 1 shows the expression of recombinant protein Mtb32a-4T by Western blot assay;
FIG. 2 shows the expression of recombinant protein Ag85a-4T, ESAT6-CFP10-4T, and RV2660-TB10.4-4T by Western blot assay;
FIG. 3 shows the results of size-exclusion chromatographic separation and purification of recombinant protein Mtb32a-4T;
FIG. 4 shows the results of size-exclusion chromatographic separation and purification of recombinant protein Ag85a-4T;
FIG. 5 shows the results of size-exclusion chromatographic separation and purification of recombinant protein ESAT6-CFP10-4T;
FIG. 6 shows the results of size-exclusion chromatographic separation and purification of recombinant protein RV2660-TB10.4-4T;
FIG. 7 shows the results of size-exclusion chromatographic separation and purification of recombinant protein M72-4T;
FIG. 8 shows the results of Octyl Bestarose 4FF separation and purification of NPM-4C;
FIG. 9 shows the results of separation and purification of recombinant protein Mtb32a-NPM binding product;
FIG. 10 shows the results of separation and purification of recombinant protein Ag85a-NPM binding product;
FIG. 11 shows the results of separation and purification of recombinant protein ESAT6-CFP10-NPM binding product;
FIG. 12 shows the results of separation and purification of recombinant protein RV2660-TB10.4-NPM binding product;
FIGs. 13-16 show the negative staining electron microscopy results of nanoparticles of recombinant proteins Mtb32a-NPM, Ag85a-NPM, ESAT6-CFP10-NPM, and RV2660-TB10.4-NPM, respectively;
FIG. 17 shows the distribution curves by intensity/volume of Mtb32a-NPM, Ag85a-NPM, ESAT6-CFP10-NPM, and RV2660-TB10.4-NPM nanoparticles;
FIG. 18 shows SDS-PAGE analyses of RV2660-TB10.4 before and after mutation;
FIG. 19 shows the distribution curves by intensity/volume of NPM nanoparticles prepared from RV2660-TB10.4 before and after mutation;
FIG. 20 shows the total bound IgG antibody levels in the serum on D20 of the corresponding groups using 072, 076, 077, 014, and M72 proteins as the coating antigen, wherein FIGs. 20a-d show the corresponding IgG antibody levels in the different groups (vac-1, vac-2, single Mtb nanoparticle antigen vaccines, and normal saline) detected by using the 072, 076, 077, and 014 antigens, respectively, as the coating antigen, and FIG. 20e shows the IgG antibody levels detected by using M72 antigen;
FIG. 21 shows the IgG1 and IgG2a antibody levels in the serum on D20 of the corresponding groups using 072, 076, 077, 014, and M72 proteins as the coating antigen, wherein FIGs. 21a-d show the corresponding IgG1 and IgG2a antibody levels in the different groups (vac-1, vac-2, single Mtb nanoparticle antigen vaccines, and normal saline) detected by using the 072, 076, 077, and 014 antigens, respectively, as the coating antigen, and FIG. 21e shows the IgG1 and IgG2a antibody levels detected of the M72 group (0.8 µg immunization group);
FIGs. 22a-d show the levels of IFN-γ, IL-2, TNFα, and IL-4, respectively, corresponding to different groups (vac-1, vac-2, single Mtb nanoparticle antigen vaccines, and normal saline) using 072 antigen as the specific antigen stimulator; FIGs. 22e-h show the levels of IFN-γ, IL-2, TNFα, and IL-4, respectively, corresponding to different groups (vac-1, vac-2, single Mtb nanoparticle antigen vaccines, and normal saline) using 076 antigen as the specific antigen stimulator;
FIGs. 23a-d show the levels of IFN-γ, IL-2, TNFα, and IL-4, respectively, corresponding to different groups (vac-1, vac-2, single Mtb nanoparticle antigen vaccines, and normal saline) using 077 antigen as the specific antigen stimulator; FIGs. 23e-h show the levels of IFN-γ, IL-2, TNFα, and IL-4, respectively, corresponding to different groups (vac-1, vac-2, single Mtb nanoparticle antigen vaccines, and normal saline) using 014 antigen as the specific antigen stimulator; and
FIG. 24 shows the detection of specific levels of IFN-γ, IL-2, TNFα, and IL-4 on D20 spleen cells using M72 protein as the stimulating antigen.

### DETAILED DESCRIPTION

The principles and features disclosed herein are described with reference to the following examples, and the examples provided are only intended to explain the present disclosure and are not intended to limit the scope disclosed herein. Before the detailed description disclosed herein is further provided, it will be appreciated that the protection scope disclosed herein is not limited to the specific embodiments described below; it will also be appreciated that the terms used in the examples herein are intended to describe specific embodiments, rather than limit the protection scope disclosed herein. Procedures without specified conditions in the following examples are generally conducted according to conventional conditions or according to conditions recommended by manufacturers. When numerical ranges are given in the examples, it will be appreciated that, unless otherwise specified in the present disclosure, both endpoints of each of the numerical ranges and any numerical value between the two endpoints can be selected. Unless otherwise defined, all technical and scientific terms used herein have the same meaning as commonly understood by those skilled in the art. In addition to the specific methods, equipment, and materials used in the examples, any methods, equipment, and materials similar or equivalent to those described in the examples herein can also be used to implement the present disclosure, based on the understanding of the prior art by those skilled in the art and the disclosure disclosed herein. The experimental materials used in the following examples were purchased from conventional reagent suppliers, unless otherwise specified.

### Example 1: Construction and expression of genes encoding M. tuberculosis structural protein - binding peptide 1 fusion protein

### 1. Construction of recombinant antigens

Mtb32a-4T, Ag85a-4T, ESAT6-CFP10-4T, and RV2660-TB10.4-4T fusion proteins were constructed by referring to the sequences of structural proteins Mtb32a, Ag85a, ESAT6, CFP10, RV2660, and TB10.4 of *M. tuberculosis* H37RV strain (GenBank: AL123456.3). M72-4T fusion protein was constructed by referring to the sequence of M72 in Patent Publication No. CN103249431B of GSK. The specific construction procedures are as follows. The specific sequences of the constructed proteins and the reference sequences described above are shown in Table 1.

### 1) Mtb32a-4T

The original wild-type sequence was subjected to S/177aa→A/177aa mutation. Linker (GSGGSG), 4T (AHIVMVDAYKPTK), and 6His (HHHHHH) sequences were sequentially added to the C-terminus in sequence, ending with a stop codon. The sequence was designated as 014.

### 2) Ag85a-4T

The original wild-type sequence was truncated at the N-terminus, and the specific sequence truncated at the N-terminus was QLVDRVRGAVTGMSRRLVVGAVGAALVSGLVGAVGGTATAG. Additionally, the linker (GSGGSG), 4T (AHIVMVDAYKPTK), and 6His (HHHHHH) sequences were sequentially added to the C-terminus of the protein in sequence, ending with a stop codon. The sequence was designated as 072.

### 3) ESAT6-CFP10-4T

The ESAT6 protein was placed at the N-terminus of the fusion protein, the CFP10 protein was placed at the C-terminus of the fusion protein, and the two were linked via a linker (GSGGSG) sequence. The linker (GSGGSG), 4T (AHIVMVDAYKPTK), and 6His (HHHHHH) sequences were sequentially added to the C-terminus in sequence, ending with a stop codon. The sequence was designated as 076.

### 4) RV2660-TB10.4-4T

The original wild-type sequence of RV2660 was subjected to C/66aa → A/66aa mutation. The mutant RV2660 protein was placed at the N-terminus of the fusion protein, the TB10.4 protein was placed at the C-terminus of the fusion protein, and the two were linked via a linker (GSGGSG) sequence. The linker (GSGGSG), 4T (AHIVMVDAYKPTK), and 6His (HHHHHH) sequences were sequentially added to the C-terminus in sequence, ending with a stop codon. The sequence was designated as 077.

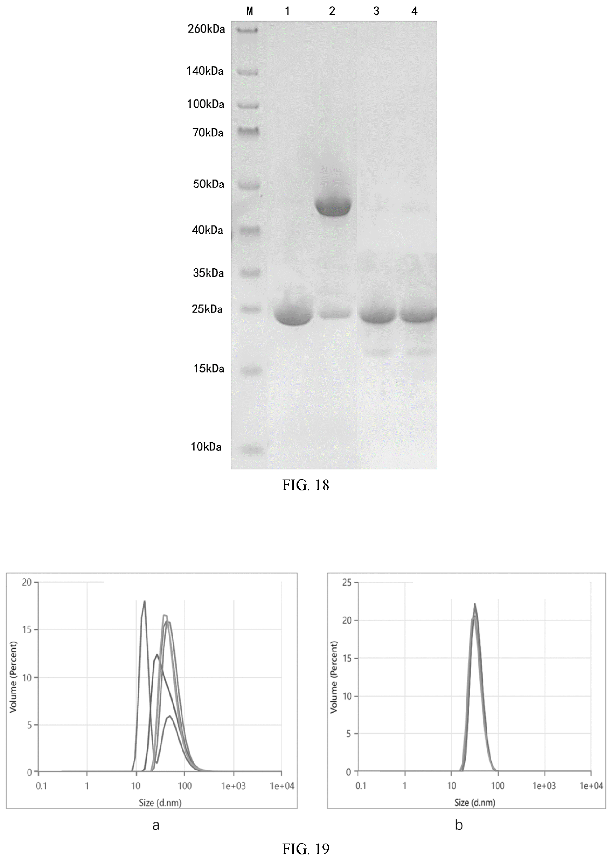

Surprisingly, it was found in the study that performing C/66aa → A/66aa on the original wild-type sequence of RV2660 can prevent the dimerization in RV2660-TB10.4-4T, such that RV2660-TB10.4-4T after mutation remained in a soluble monomeric form (a single band in both reduced and non-reduced states according to SDS-PAGE analysis). The details are shown in FIG. 18. The mutation made the formed RV2660-TB10.4-NPM nanoparticles more homogeneous (Z-average and polydispersity index values changed from 77.3 and 0.23 before the mutation to 39.8 and 0.14 after the mutation, respectively). The details are shown in FIG. 19.

In FIG. 18, M: protein molecule marker, lane 1: RV2660-TB10.4-4T before mutation (DTT+), lane 2: RV2660-TB10.4-4T mutation (DTT-), lane 3: RV2660-TB10.4-4T after mutation (DTT+), lane 4: RV2660-TB10.4-4T after mutation (DTT-).

In FIG. 19, panel a shows an NPM nanoparticle prepared from RV2660-TB10.4 before mutation; panel b shows an NPM nanoparticle prepared from RV2660-TB10.4 after mutation.

### 5) M72-4T

Referring to the sequence of the M72 fusion protein in the patent published by GSK (granted publication number CN 103249431B), the 6His (HHHHHH), 4T (AHIVMVDAYKPTK), and linker (GSGGSG) sequences were added sequentially at the N-terminus, ending with a stop codon.

The protein sequences designed above (see Table 1 for the sequences) were subjected to *E. coli* codon optimization and gene synthesis (see Table 2 below for the sequences), cloned into pET21a vectors at enzyme cleavage sites (5' NdeI and 3' HindIII), and transformed into BL21(DE3) for downstream expression.

**Table 1: Amino acid sequences of Mtb32a-4T, Ag85a-4T, ESAT6-CFP10-4T, RV2660-TB10.4-4T, M72-4T, binding peptide 1, linker peptide 1, and fusion protein**

| Polypeptide name | Amino acid sequence |
|---|---|
| Binding peptide 1 (4T) | AHIVMVDAYKPTK (SEQ ID NO:1) |
| Linker peptide 1 | GSGGSG (SEQ ID NO:2) |
| Mtb32a | MAPPALSQDRFADFPALPLDPSAMVAQVGPQVVNINTKLGYNNAVGAGTGIVIDPNGVVLTNN HVIAGATDINAFSVGSGQTYGVDVVGYDRTQDVAVLQLRGAGGLPSAAIGGGVAVGEPVVAM GNSGGQGGTPRAVPGRVVALGQTVQASDSLTGAEETLNGLIQFDAAIQPGDAGGPVVNGLGQV VGMNTAASDNFQLSQGGQGFAIPIGQAMAIAGQIRSGGGSPTVHIGPTAFLGLGVVDNNGNGA RVQRVVGSAPAASLGISTGDVITAVDGAPINSATAMADALNGHHPGDVISVTWQTKSGGTRTG NVTLAEGPPA (SEQ ID NO:3) |
| Mtb32a-linker peptide 1-binding | MAPPALSQDRFADFPALPLDPSAMVAQVGPQVVNINTKLGYNNAVGAGTGIVIDPNGVVLTNN HVIAGATDINAFSVGSGQTYGVDVVGYDRTQDVAVLQLRGAGGLPSAAIGGGVAVGEPVVAM |
| peptide 1 (Mtb32a-4T) | GNSGGQGGTPRAVPGRVVALGQTVQASDSLTGAEETLNGLIQFDAAIQPGDAGGPVVNGLGQV VGMNTAASDNFQLSQGGQGFAIPIGQAMAIAGQIRSGGGSPTVHIGPTAFLGLGVVDNNGNGA RVQRVVGSAPAASLGISTGDVITAVDGAPINSATAMADALNGHHPGDVISVTWQTKSGGTRTG NVTLAEGPPAGSGGSGAHIVMVDAYKPTKHHHHHH (SEQ ID NO:4) |
| Ag85a | MAFSRPGLPVEYLQVPSPSMGRDIKVQFQSGGANSPALYLLDGLRAQDDFSGWDINTPAFEWY DQSGLSVVMPVGGQSSFYSDWYQPACGKAGCQTYKWETFLTSELPGWLQANRHVKPTGSAV VGLSMAASSALTLAIYHPQQFVYAGAMSGLLDPSQAMGPTLIGLAMGDAGGYKASDMWGPK EDPAWQRNDPLLNVGKLIANNTRVWVYCGNGKPSDLGGNNLPAKFLEGFVRTSNIKFQDAYN AGGGHNGVFDFPDSGTHSWEYWGAQLNAMKPDLQRALGATPNTGPAPQGA (SEQ ID NO:5) |
| Ag85a-linker peptide 1-binding peptide 1 (Ag85a-4T) | MAFSRPGLPVEYLQVPSPSMGRDIKVQFQSGGANSPALYLLDGLRAQDDFSGWDINTPAFEWY DQSGLSVVMPVGGQSSFYSDWYQPACGKAGCQTYKWETFLTSELPGWLQANRHVKPTGSAV VGLSMAASSALTLAIYHPQQFVYAGAMSGLLDPSQAMGPTLIGLAMGDAGGYKASDMWGPK EDPAWQRNDPLLNVGKLIANNTRVWVYCGNGKPSDLGGNNLPAKFLEGFVRTSNIKFQDAYN AGGGHNGVFDFPDSGTHSWEYWGAQLNAMKPDLQRALGATPNTGPAPQGAGSGGSGAHIVM VDAYKPTKHHHHHH (SEQ ID NO:6) |
| ESAT6-CFP10 | MTEQQWNFAGIEAAASAIQGNVTSIHSLLDEGKQSLTKLAAAWGGSGSEAYQGVQQKWDATA TELNNALQNLARTISEAGQAMASTEGNVTGMFAGSGGSGAEMKTDAATLAQEAGNFERISGDL KTQIDQVESTAGSLQGQWRGAAGTAAQAAVVRFQEAANKQKQELDEISTNIRQAGVQYSRAD EEQQQALSSQMGF (SEQ ID NO:7) |
| ESAT6-CFP10-linker peptide 1-binding peptide 1 (ESAT6-CFP10-4T) | MTEQQWNFAGIEAAASAIQGNVTSIHSLLDEGKQSLTKLAAAWGGSGSEAYQGVQQKWDATA TELNNALQNLARTISEAGQAMASTEGNVTGMFAGSGGSGAEMKTDAATLAQEAGNFERISGDL KTQIDQVESTAGSLQGQWRGAAGTAAQAAVVRFQEAANKQKQELDEISTNIRQAGVQYSRAD EEQQQALSSQMGFGSGGSGAHIVMVDAYKPTKHHHHHH (SEQ ID NO:8) |
| RV2660-TB10.4 | MIAGVDQALAATGQASQRAAGASGGVTVGVGVGTEQRNLSVVAPSQFTFSSRSPDFVDETAG QSWAAILGLNQFHGSGGSGSQIMYNYPAMLGHAGDMAGYAGTLQSLGAEIAVEQAALQSAW QGDTGITYQAWQAQWNQAMEDLVRAYHAMSSTHEANTMAMMARDTAEAAKWGG (SEQ ID NO:9) |
| RV2660-TB10.4-linker peptide 1-binding peptide 1 (RV2660-TB10.4-4T) | MIAGVDQALAATGQASQRAAGASGGVTVGVGVGTEQRNLSVVAPSQFTFSSRSPDFVDETAG QSWAAILGLNQFHGSGGSGSQIMYNYPAMLGHAGDMAGYAGTLQSLGAEIAVEQAALQSAW QGDTGITYQAWQAQWNQAMEDLVRAYHAMSSTHEANTMAMMARDTAEAAKWGGGSGGSG AHIVMVDAYKPTKHHHHHH (SEQ ID NO:10) |
| M72-linker peptide 1-binding peptide 1 (M72-4T) | MHHHHHHAHIVMVDAYKPTKGSGGSGTAASDNFQLSQGGQGFAIPIGQAMAIAGQIRSGGGSP TVHIGPTAFLGLGVVDNNGNGARVQRVVGSAPAASLGISTGDVITAVDGAPINSATAMADALN GHHPGDVISVTWQTKSGGTRTGNVTLAEGPPAEFMVDFGALPPEINSARMYAGPGSASLVAAA QMWDSVASDLFSAASAFQSVVWGLTVGSWIGSSAGLMVAAASPYVAWMSVTAGQAELTAAQ VRVAAAAYETAYGLTVPPPVIAENRAELMILIATNLLGQNTPAIAVNEAEYGEMWAQDAAAMF GYAAATATATATLLPFEEAPEMTSAGGLLEQAAAVEEASDTAAANQLMNNVPQALQQLAQPT QGTTPSSKLGGLWKTVSPHRSPISNMVSMANNHMSMTNSGVSMTNTLSSMLKGFAPAAAAQA VQTAAQNGVRAMSSLGSSLGSSGLGGGVAANLGRAASVGSLSVPQAWAAANQAVTPAARAL PLTSLTSAAERGPGQMLGGLPVGQMGARAGGGLSGVLRVPPRPYVMPHSPAAGDIAPPALSQD |
| | RFADFPALPLDPSAMVAQVGPQVVNINTKLGYNNAVGAGTGIVIDPNGVVLTNNHVIAGATDI NAFSVGSGQTYGVDVVGYDRTQDVAVLQLRGAGGLPSAAIGGGVAVGEPVVAMGNSGGQGG TPRAVPGRVVALGQTVQASDSLTGAEETLNGLIQFDAAIQPGDAGGPVVNGLGQVVGMNTAAS (SEQ ID NO:11) |

**Table 2: Nucleotide sequences of Mtb32a-4T, Ag85a-4T, ESAT6-CFP10-4T, RV2660-TB10.4-4T, M72-4T, binding peptide 1, linker peptide 1, and fusion protein**

| Polypeptide name | Nucleotide sequence |
|---|---|
| Binding peptide 1 (4T) | GCCCACATTGTTATGGTGGACGCATACAAACCGACCAAA (SEQ ID NO:12) |
| Linker peptide 1 | GGTTCCGGCGGTTCTGGT (SEQ ID NO:13) |
| 6His | CACCACCACCATCACCAC (SEQ ID NO:14) |
| Mtb32a | ATGGCTCCGCCTGCACTGTCCCAGGACCGTTTCGCTGACTTCCCTGCTCTGCCGCTGGATCCG TCTGCGATGGTGGCTCAGGTCGGCCCGCAGGTGGTCAACATTAACACCAAACTGGGCTATA ACAACGCTGTTGGTGCGGGTACTGGTATCGTGATCGATCCGAACGGTGTTGTGCTGACTAAC AACCACGTGATCGCTGGCGCTACGGACATCAACGCCTTCTCCGTCGGTAGCGGTCAAACCTA TGGTGTCGACGTGGTCGGCTACGATCGTACCCAGGACGTTGCTGTTCTGCAGCTGCGTGGTG CTGGCGGCCTGCCGTCTGCCGCTATTGGTGGTGGTGTGGCAGTAGGTGAGCCAGTGGTGGCT ATGGGCAACTCTGGTGGCCAGGGTGGCACTCCTCGTGCGGTACCAGGCCGTGTCGTGGCTCT GGGCCAGACTGTTCAGGCCAGCGACAGCCTGACTGGCGCGGAAGAGACTCTGAATGGCCTG ATCCAGTTCGACGCTGCAATCCAACCGGGCGATGCGGGCGGTCCTGTAGTAAACGGCCTGG GTCAGGTAGTGGGTATGAACACCGCTGCTAGCGATAATTTCCAACTGAGCCAGGGCGGCCA GGGTTTCGCAATCCCTATTGGCCAGGCCATGGCAATCGCAGGCCAGATCCGTAGCGGTGGT GGCTCCCCGACGGTACATATCGGTCCGACCGCGTTCCTGGGCCTGGGCGTGGTGGACAATA ACGGCAACGGTGCGCGTGTGCAACGTGTAGTCGGCTCCGCTCCAGCAGCTTCCCTGGGTATC AGCACTGGTGACGTCATTACTGCTGTCGATGGTGCGCCGATCAACTCTGCTACTGCGATGGC AGATGCACTGAACGGTCACCACCCTGGCGACGTTATCTCCGTTACGTGGCAGACGAAATCC GGTGGCACCCGTACGGGCAACGTGACCCTGGCGGAAGGCCCGCCGGCA (SEQ ID NO:15) |
| Mtb32a-linker peptide 1-binding peptide 1 (Mtb32a-4T) | ATGGCTCCGCCTGCACTGTCCCAGGACCGTTTCGCTGACTTCCCTGCTCTGCCGCTGGATCCG TCTGCGATGGTGGCTCAGGTCGGCCCGCAGGTGGTCAACATTAACACCAAACTGGGCTATA ACAACGCTGTTGGTGCGGGTACTGGTATCGTGATCGATCCGAACGGTGTTGTGCTGACTAAC AACCACGTGATCGCTGGCGCTACGGACATCAACGCCTTCTCCGTCGGTAGCGGTCAAACCTA TGGTGTCGACGTGGTCGGCTACGATCGTACCCAGGACGTTGCTGTTCTGCAGCTGCGTGGTG CTGGCGGCCTGCCGTCTGCCGCTATTGGTGGTGGTGTGGCAGTAGGTGAGCCAGTGGTGGCT ATGGGCAACTCTGGTGGCCAGGGTGGCACTCCTCGTGCGGTACCAGGCCGTGTCGTGGCTCT GGGCCAGACTGTTCAGGCCAGCGACAGCCTGACTGGCGCGGAAGAGACTCTGAATGGCCTG ATCCAGTTCGACGCTGCAATCCAACCGGGCGATGCGGGCGGTCCTGTAGTAAACGGCCTGG GTCAGGTAGTGGGTATGAACACCGCTGCTAGCGATAATTTCCAACTGAGCCAGGGCGGCCA GGGTTTCGCAATCCCTATTGGCCAGGCCATGGCAATCGCAGGCCAGATCCGTAGCGGTGGT GGCTCCCCGACGGTACATATCGGTCCGACCGCGTTCCTGGGCCTGGGCGTGGTGGACAATA ACGGCAACGGTGCGCGTGTGCAACGTGTAGTCGGCTCCGCTCCAGCAGCTTCCCTGGGTATC AGCACTGGTGACGTCATTACTGCTGTCGATGGTGCGCCGATCAACTCTGCTACTGCGATGGC |
| | AGATGCACTGAACGGTCACCACCCTGGCGACGTTATCTCCGTTACGTGGCAGACGAAATCC GGTGGCACCCGTACGGGCAACGTGACCCTGGCGGAAGGCCCGCCGGCAGGTTCCGGCGGTT CTGGTGCCCACATTGTTATGGTGGACGCATACAAACCGACCAAACACCAACCATCACCAC (SEQ ID NO:16) |
| Ag85a | ATGGCCTTCTCCCGCCCTGGTCTGCCGGTCGAATATCTGCAAGTTCCGAGCCCGTCTATGGG TCGCGACATTAAAGTTCAGTTCCAGTCCGGCGGCGCAAACTCCCCTGCGCTGTATCTGCTGG ATGGTCTGCGCGCACAGGACGACTTTTCCGGCTGGGATATCAACACCCCGGCATTCGAGTGG TATGACCAGTCCGGTCTGTCCGTTGTTATGCCGGTTGGCGGTCAGTCCTCCTTCTATTCTGAC TGGTACCAGCCGGCCTGCGGCAAAGCTGGTTGCCAGACGTACAAATGGGAAACCTTCCTGA CGTCCGAACTGCCAGGCTGGCTGCAGGCGAACCGTCACGTCAAGCCGACCGGTTCTGCAGT AGTAGGTCTGTCTATGGCTGCCTCCTCTGCTCTGACTCTGGCTATCTACCACCCGCAGCAATT CGTGTACGCGGGTGCGATGAGCGGTCTGCTGGACCCGAGCCAAGCTATGGGTCCAACCCTG ATCGGTCTGGCAATGGGCGATGCGGGTGGCTATAAAGCTTCCGACATGTGGGGCCCAAAAG AAGATCCAGCCTGGCAACGTAACGACCCGCTGCTGAACGTCGGCAAACTGATCGCTAACAA CACCCGCGTATGGGTATACTGTGGTAACGGTAAGCCGAGCGACCTGGGTGGTAACAACCTG CCGGCGAAATTCCTGGAAGGTTTTGTGCGTACGAGCAACATCAAGTTCCAAGACGCGTATA ACGCTGGCGGCGGTCACAACGGTGTCTTCGATTTCCCGGACTCCGGCACCCATTCCTGGGAA TATTGGGGCGCCCAGCTGAACGCTATGAAACCTGATCTGCAACGTGCCCTGGGCGCAACGC CAAACACCGGTCCAGCTCCGCAGGGTGCT (SEQ ID NO:17) |
| Ag85a-linker peptide 1-binding peptide 1 (Ag85a-4T) | ATGGCCTTCTCCCGCCCTGGTCTGCCGGTCGAATATCTGCAAGTTCCGAGCCCGTCTATGGG TCGCGACATTAAAGTTCAGTTCCAGTCCGGCGGCGCAAACTCCCCTGCGCTGTATCTGCTGG ATGGTCTGCGCGCACAGGACGACTTTTCCGGCTGGGATATCAACACCCCGGCATTCGAGTGG TATGACCAGTCCGGTCTGTCCGTTGTTATGCCGGTTGGCGGTCAGTCCTCCTTCTATTCTGAC TGGTACCAGCCGGCCTGCGGCAAAGCTGGTTGCCAGACGTACAAATGGGAAACCTTCCTGA CGTCCGAACTGCCAGGCTGGCTGCAGGCGAACCGTCACGTCAAGCCGACCGGTTCTGCAGT AGTAGGTCTGTCTATGGCTGCCTCCTCTGCTCTGACTCTGGCTATCTACCACCCGCAGCAATT CGTGTACGCGGGTGCGATGAGCGGTCTGCTGGACCCGAGCCAAGCTATGGGTCCAACCCTG ATCGGTCTGGCAATGGGCGATGCGGGTGGCTATAAAGCTTCCGACATGTGGGGCCCAAAAG AAGATCCAGCCTGGCAACGTAACGACCCGCTGCTGAACGTCGGCAAACTGATCGCTAACAA CACCCGCGTATGGGTATACTGTGGTAACGGTAAGCCGAGCGACCTGGGTGGTAACAACCTG CCGGCGAAATTCCTGGAAGGTTTTGTGCGTACGAGCAACATCAAGTTCCAAGACGCGTATA ACGCTGGCGGCGGTCACAACGGTGTCTTCGATTTCCCGGACTCCGGCACCCATTCCTGGGAA TATTGGGGCGCCCAGCTGAACGCTATGAAACCTGATCTGCAACGTGCCCTGGGCGCAACGC CAAACACCGGTCCAGCTCCGCAGGGTGCTGGTTCCGGCGGTTCTGGTGCCCACATTGTTATG GTGGACGCATACAAACCGACCAAACACCACCACCATCACCAC (SEQ ID NO:18) |
| ESAT6-CFP10 | ATGACCGAACAGCAGTGGAACTTCGCGGGCATTGAAGCAGCAGCATCTGCAATCCAGGGCA ACGTTACTTCCATTCACTCTCTGCTGGACGAGGGTAAGCAGTCTCTGACCAAACTGGCAGCG GCGTGGGGCGGTTCTGGTTCTGAGGCTTACCAGGGCGTGCAGCAGAAATGGGACGCGACTG CGACTGAGCTGAACAACGCTCTGCAGAACCTGGCTCGTACTATCAGCGAGGCCGGTCAAGC GATGGCGTCTACGGAAGGTAACGTGACCGGCATGTTCGCGGGTTCCGGTGGTAGCGGTGCG GAGATGAAAACCGATGCCGCGACTCTGGCACAGGAAGCTGGTAACTTCGAACGCATTAGCG GTGACCTGAAGACCCAGATCGATCAGGTCGAATCCACCGCCGGCAGCCTGCAAGGTCAGTG GCGTGGCGCTGCAGGCACGGCGGCCCAAGCAGCTGTGGTTCGTTTCCAGGAAGCTGCGAAC |
| | AAACAGAAACAGGAGCTGGACGAGATCTCTACGAACATCCGTCAGGCTGGTGTGCAGTATT CCCGTGCAGACGAAGAACAGCAACAGGCGCTGTCTAGCCAGATGGGCTTT (SEQ ID NO:19) |
| ESAT6-CFP10-linker peptide 1-binding peptide 1 (ESAT6-CFP10-4T) | ATGACCGAACAGCAGTGGAACTTCGCGGGCATTGAAGCAGCATCTGCAATCCAGGGCA ACGTTACTTCCATTCACTCTCTGCTGGACGAGGGTAAGCAGTCTCTGACCAAACTGGCAGCG GCGTGGGGCGGTTCTGGTTCTGAGGCTTACCAGGGCGTGCAGCAGAAATGGGACGCGACTG CGACTGAGCTGAACAACGCTCTGCAGAACCTGGCTCGTACTATCAGCGAGGCCGGTCAAGC GATGGCGTCTACGGAAGGTAACGTGACCGGCATGTTCGCGGGTTCCGGTGGTAGCGGTGCG GAGATGAAAACCGATGCCGCGACTCTGGCACAGGAAGCTGGTAACTTCGAACGCATTAGCG GTGACCTGAAGACCCAGATCGATCAGGTCGAATCCACCGCCGGCAGCCTGCAAGGTCAGTG GCGTGGCGCTGCAGGCACGGCGGCCCAAGCAGCTGTGGTTCGTTTCCAGGAAGCTGCGAAC AAACAGAAACAGGAGCTGGACGAGATCTCTACGAACATCCGTCAGGCTGGTGTGCAGTATT CCCGTGCAGACGAAGAACAGCAACAGGCGCTGTCTAGCCAGATGGGCTTTGGTTCCGGCGG TTCTGGTGCCCACATTGTTATGGTGGACGCATACAAACCGACCAAA CACCACCACCATCACCAC (SEQ ID NO:20) |
| RV2660-TB10.4 | ATGATTGCAGGCGTGGATCAGGCACTGGCAGCGACCGGTCAGGCATCTCAGCGTGCGGCCG GCGCCAGCGGCGGTGTTACGGTTGGCGTTGGCGTGGGTACCGAACAGCGCAACCTGTCCGT GGTGGCTCCGTCTCAATTCACGTTCAGCTCCCGCTCCCCGGATTTTGTCGACGAAACTGCGG GTCAGAGCTGGGCTGCTATCCTGGGCCTGAACCAGTTTCACGGCTCCGGTGGCAGCGGCTCC CAGATCATGTACAACTACCCGGCCATGCTGGGTCATGCGGGTGATATGGCAGGCTACGCAG GTACTCTGCAGTCTCTGGGCGCGGAAATCGCGGTTGAACAGGCTGCACTGCAGTCTGCATGG CAGGGTGACACCGGCATCACGTATCAGGCTTGGCAGGCGCAGTGGAATCAGGCCATGGAAG ACCTGGTACGTGCGTACCACGCGATGTCCTCTACGCATGAAGCGAACACCATGGCCATGAT GGCTCGTGACACCGCAGAAGCAGCGAAATGGGGCGGC (SEQ ID NO:21) |
| RV2660-TB10.4-linker peptide 1-binding peptide 1 (RV2660-TB10.4-4T) | ATGATTGCAGGCGTGGATCAGGCACTGGCAGCGACCGGTCAGGCATCTCAGCGTGCGGCCG GCGCCAGCGGCGGTGTTACGGTTGGCGTTGGCGTGGGTACCGAACAGCGCAACCTGTCCGT GGTGGCTCCGTCTCAATTCACGTTCAGCTCCCGCTCCCCGGATTTTGTCGACGAAACTGCGG GTCAGAGCTGGGCTGCTATCCTGGGCCTGAACCAGTTTCACGGCTCCGGTGGCAGCGGCTCC CAGATCATGTACAACTACCCGGCCATGCTGGGTCATGCGGGTGATATGGCAGGCTACGCAG GTACTCTGCAGTCTCTGGGCGCGGAAATCGCGGTTGAACAGGCTGCACTGCAGTCTGCATGG CAGGGTGACACCGGCATCACGTATCAGGCTTGGCAGGCGCAGTGGAATCAGGCCATGGAAG ACCTGGTACGTGCGTACCACGCGATGTCCTCTACGCATGAAGCGAACACCATGGCCATGAT GGCTCGTGACACCGCAGAAGCAGCGAAATGGGGCGGCGGTTCCGGCGGTTCTGGTGCCCAC ATTGTTATGGTGGACGCATACAAACCGACCAAA CACCACCACCATCACCAC (SEQ ID NO:22) |
| M72-linker peptide 1-binding peptide 1 (M72-4T) | ATGCACCACCACCATCACCAC GCCCACATTGTTATGGTGGACGCATACAAAC CGACCAAA GGTTCCGGCGGTTCTGGT ACTGCCGCGTCCGATAACTTTCAG CTGTCCCAAGGTGGTCAAGGTTTCGCGATTCCGATTGGCCAAGCGATGGCTATCGCAGGCCA AATCCGTTCCGGTGGCGGTTCCCCGACTGTCCACATCGGTCCGACCGCTTTCCTGGGCCTGG GCGTGGTTGACAACAATGGTAACGGTGCACGCGTTCAGCGCGTTGTTGGCTCCGCGCCTGCA GCGAGCCTGGGCATCAGCACCGGCGATGTAATTACCGCAGTTGATGGCGCGCCGATCAATT CTGCCACCGCTATGGCTGACGCGCTGAACGGTCACCACCCGGGCGACGTCATCTCCGTTACT TGGCAGACTAAAAGCGGTGGTACCCGTACCGGCAACGTTACCCTGGCGGAAGGTCCACCGG CGGAATTCATGGTTGACTTCGGTGCACTGCCGCCGGAAATCAATAGCGCCCGCATGTACGCA GGTCCGGGTTCCGCGTCTCTGGTTGCGGCAGCCCAGATGTGGGACTCTGTTGCGAGCGACCT |
| | GTTCTCCGCGGCCTCCGCATTCCAGTCCGTAGTTTGGGGTCTGACGGTTGGTTCCTGGATCG GTTCCTCTGCGGGCCTGATGGTCGCTGCTGCGAGCCCGTACGTTGCGTGGATGTCCGTTACC GCTGGTCAGGCCGAACTGACCGCGGCTCAGGTCCGTGTAGCGGCGGCATACGAGACCG CCTACGGCCTGACTGTGCCGCCACCTGTCATCGCAGAGAATCGTGCGGAGCTGATGATTCTG ATCGCGACGAACCTGCTGGGTCAGAATACTCCGGCTATCGCTGTCAACGAGGCGGAATACG GCGAAATGTGGGCTCAGGACGCAGCCGCAATGTTTGGTTATGCTGCTGCAACCGCGACCGC CACCGCTACCCTGCTGCCGTTCGAAGAGGCCCCGGAAATGACGTCTGCAGGTGGTCTGCTGG AACAAGCTGCTGCCGTGGAGGAGGCGTCTGACACCGCGGCTGCTAATCAGCTGATGAACAA CGTGCCGCAGGCACTGCAGCAGCTGGCGCAGCCGACTCAAGGCACGACCCCGTCTTCTAAG CTGGGCGGTCTGTGGAAAACCGTATCCCCGCATCGTAGCCCGATTAGCAACATGGTTAGCAT GGCGAACAACCATATGTCCATGACTAACTCCGGCGTGAGCATGACCAACACCCTGTCCTCTA TGCTGAAAGGTTTCGCCCCGGCGGCTGCCGCCCAAGCTGTCCAGACCGCAGCTCAGAACGG CGTTCGTGCGATGTCCTCCCTGGGCTCTTCCCTGGGCTCTTCTGGCCTGGGTGGTGGCGTTGC TGCTAATCTGGGCCGCGCAGCAAGCGTCGGTAGCCTGTCTGTCCCGCAGGCATGGGCAGCC GCGAACCAGGCTGTGACTCCTGCCGCACGCGCTCTGCCGCTGACTTCCCTGACGTCTGCCGC AGAACGTGGTCCGGGCCAGATGCTGGGTGGTCTGCCAGTAGGTCAGATGGGTGCCCGTGCG GGTGGTGGTCTGTCCGGTGTTCTGCGTGTACCACCGCGTCCGTACGTCATGCCGCACTCCCC GGCCGCGGGTGACATCGCCCCACCGGCGCTGAGCCAAGACCGCTTCGCGGACTTTCCTGCTC TGCCGCTGGACCCGTCCGCTATGGTTGCACAAGTGGGTCCGCAGGTGGTGAACATCAACAC CAAACTGGGTTACAACAACGCTGTTGGTGCTGGTACTGGTATCGTAATCGACCCGAACGGC GTGGTACTGACCAACAACCACGTTATCGCCGGTGCCACCGATATCAACGCTTTCTCTGTAGG CAGCGGTCAGACCTACGGCGTGGATGTTGTTGGCTACGATCGCACCCAGGACGTTGCCGTTC TGCAGCTGCGCGGTGCTGGTGGTCTGCCGTCTGCTGCCATCGGCGGCGGCGTTGCCGTGGGC GAACCGGTAGTAGCAATGGGCAACTCCGGCGGTCAGGGTGGCACTCCTCGTGCCGTGCCGG GCCGCGTAGTAGCTCTGGGTCAGACGGTTCAGGCTAGCGATTCTCTGACCGGCGCAGAAGA AACCCTGAACGGCCTGATTCAGTTCGACGCTGCGATTCAACCGGGTGATGCGGGCGGTCCG GTAGTGAACGGCCTGGGCCAGGTGGTGGGCATGAACACCGCTGCAAGC (SEQ ID NO:23) |

### 2. Expression of recombinant antigens

BL21(DE3) expressing bacteria of Mtb32a-4T, Ag85a-4T, ESAT6-CFP10-4T, and RV2660-TB10.4-4T were inoculated onto an LB agar plate (containing 50 µg/mL of ampicillin) by streaking, and cultured overnight at 37 °C. Then single clones were picked and inoculated into 10 mL of TB medium containing 50 µg/mL of ampicillin, and cultured overnight at 37 °C and 220 rpm/min.

The bacterial solution was inoculated into a TB culture medium containing 50 µg/mL of ampicillin at a ratio of 1/100 and cultured at 37 °C and 220 rpm/min for 2-3 h.

The OD600 of the bacterial solution was determined by Nanodrop. When the OD value reached 0.6-0.8, the bacterial solution was transferred to a shaker at 18 °C and cooled, and IPTG was added overnight to induce the expression for about 16 h, with the final concentration of IPTG being 500 µM.

The bacterial solution was collected and centrifuged at 6000 g at 4 °C for 15 min, and the supernatant was discarded.

The pellet was centrifuged again at 6000 g at 4 °C for 3 min, and the medium in the supernatant was removed. The bacterial sludge was cryopreserved in a freezer at -80 °C.

### Example 2: Western blot assay of recombinant antigens

1) Mtb32a-4T, Ag85a-4T, ESAT6-CFP10-4T, and RV2660-TB10.4-4T expression bacterial sludges were resuspended in 20 mL of buffer containing 20 mM Tris-HCl, 150 mM NaCl, pH 7.4. After ultrasonication, the suspension was centrifuged at 13,000 g/min for 30 min at 4 °C, and the supernatant was collected and precipitated (suspended in a buffer containing 20 mM Tris-HCl and 150 mM NaCl at pH 7.4).
2) Western blot assay was performed by adding a reducing agent DTT to the LDS sample loading buffer (4×). The sample loading buffer was heated at 70 °C for 5 min, cooled to room temperature, centrifuged at 10,000 rpm for 20 s, and thoroughly mixed by vortex, and the final loading amount was 0.5 µg.
3) Both the test sample and the prestained protein molecular weight standard were loaded onto a 4-12% Bis-Tris gel, and the MES running buffer was applied. Electrophoresis was performed at a voltage of 150 V for approximately 60 min.
4) Membrane transfer was performed using the Trans-Blot^{®} Turbo instrument and associated reagents. The membrane was incubated with an anti-His mouse monoclonal antibody and an alkaline phosphatase (AP)-conjugated goat anti-mouse secondary antibody using an iBind instrument. Finally, a chromogenic solution was used for color development and GelDoc Go was used for photographing.

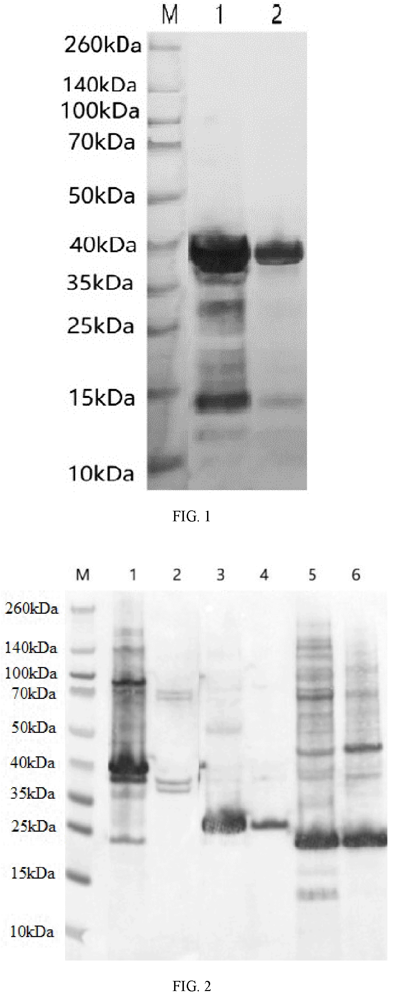

The Western blot assay results showed that all the 4 proteins were capable of soluble expression and inclusion body expression, and the band positions were consistent with the expected molecular weights, as shown in FIG. 1 and FIG. 2.

In FIG. 1: M denotes the protein molecule marker, lane 1 denotes Mtb32a-4T supernatant, and lane 2 denotes Mtb32a-4T pellet;

In FIG. 2: M denotes the protein molecule marker, lane 1 denotes Ag85a-4T supernatant, lane 2 denotes Ag85a-4T pellet, lane 3 denotes ESAT6-CFP10-4T supernatant, lane 4 denotes ESAT6-CFP10-4T pellet, lane 5 denotes RV2660-TB10.4-4T supernatant, and lane 6 denotes RV2660-TB10.4-4T pellet.

### Example 3: Purification of recombinant antigen and SDS-PAGE analysis of purified antigen

1. The antigenic components of fusion proteins Mtb32a-4T, Ag85a-4T, ESAT6-CFP10-4T, and RV2660-TB10.4-4T obtained by *E. coli* expression were purified by nickel column affinity chromatography and size-exclusion chromatography to obtain high-purity proteins. The specific procedures are as follows:

### 1) Treatment before purification

Mtb32a-4T, Ag85a-4T, ESAT6-CFP10-4T, and RV2660-TB10.4-4T expression bacterial sludges (corresponding to 200 mL of the expression bacterial solution) were resuspended in 20 mL of buffer containing 20 mM Tris-HCl, 150 mM NaCl, pH 7.4. The cells were lysed by ultrasonication and centrifuged at 13,000 g/min for 30 min at 4 °C. The pellet was discarded, and the supernatant was retained.

### 2) Nickel ion affinity chromatography

Affinity purification was accomplished using a nickel ion affinity packing chromatography column. The volume of the chromatography column was 10 mL, the flow rate for chromatography was 5 mL/min, and 70 mL of sample was loaded.

Chromatographic procedure: The Ni-Bestarose Fast Flow column was sterilized and equilibrated with a capture buffer containing 20 mM Tris-HCl, 150 mM NaCl, pH 7.4. The sample was loaded, and column was washed with a solution containing 20 mM Tris-HCl, 150 mM NaCl, pH 7.4, washed with a solution containing 20 mM Tris-HCl, 150 mM NaCl, 2% Triton-X100, pH 7.4 to remove endotoxins, washed with a buffer containing 20 mM imidazole + 20 mM Tris-HCl, 150 mM NaCl, pH 7.4 to remove protein impurities, and eluted with a buffer containing 500 mM imidazole + 20 mM Tris-HCl, 150 mM NaCl, pH 7.4 to harvest Mtb32a-4T, Ag85a-4T, ESAT6-CFP10-4T, and RV2660-TB10.4-4T antigenic components.

### 3) Size-exclusion chromatographic purification

Purification was performed using a HiLoad 16/600 Superdex 200 pg column. The column volume of the SEC column was 120 mL, and the loading amount of affinity-purified samples of MTB32A, AG85A, ESAT6-CFP10, and RV2660-TB10.4 was controlled at approximately 4% of the column volume.

Chromatographic procedure: The Superdex 200 pg was sterilized and equilibrated with an equilibration buffer (20 mM Tris-HCl, 150 mM NaCl, pH 7.4). The sample was loaded, and the column was washed with TBS solution. Mtb32a-4T, Ag85a-4T, ESAT6-CFP10-4T, and RV2660-TB10.4-4T antigenic components were collected.

### 2. SDS-PAGE analysis of purified antigens

The purified Mtb32a-4T, Ag85a-4T, ESAT6-CFP10-4T, and RV2660-TB10.4-4T antigens were subjected to SDS-PAGE analysis by adding a reducing agent DTT to the LDS sample loading buffer (4×). The sample loading buffer was heated at 70 °C for 5 min, cooled to room temperature, centrifuged at 10,000 rpm for 20 s, and thoroughly mixed by vortex, and the final loading amount was 5 µg. Both the test sample and the non-prestained protein molecular weight standard were loaded onto a 4-12% Bis-Tris gel, and the MES running buffer was applied. Electrophoresis was performed at a voltage of 150 V for approximately 60 min. After electrophoresis, the gel was removed and placed in a clean container. An appropriate amount of Coomassie brilliant blue staining solution was added to submerge the gel, followed by staining for 2 h on a shaker. After staining, the staining solution was discarded, and the gel was soaked in purified water for destaining on a shaker until the background was clear. The gel was then photographed using a GelDoc Go gel imager.

### Results and analysis:

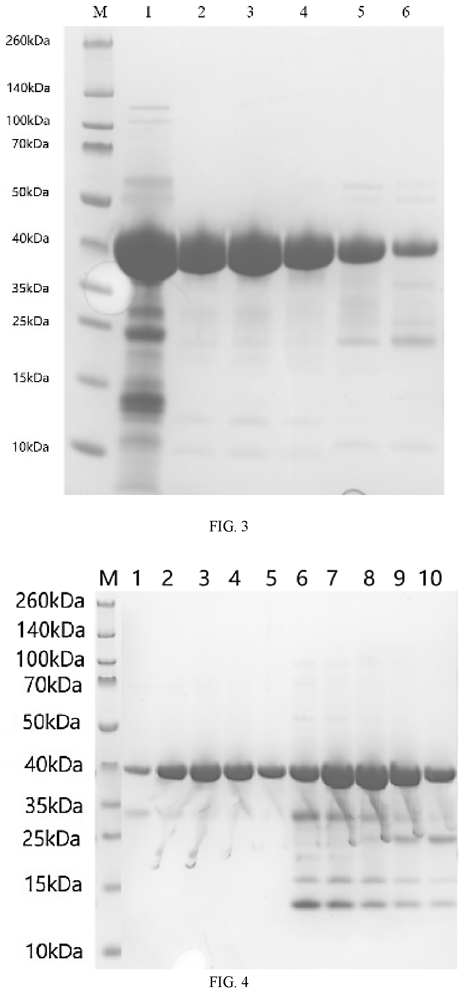

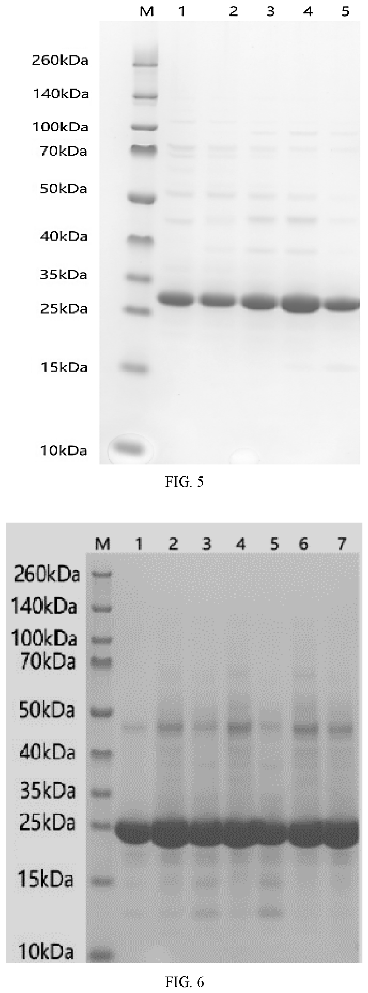

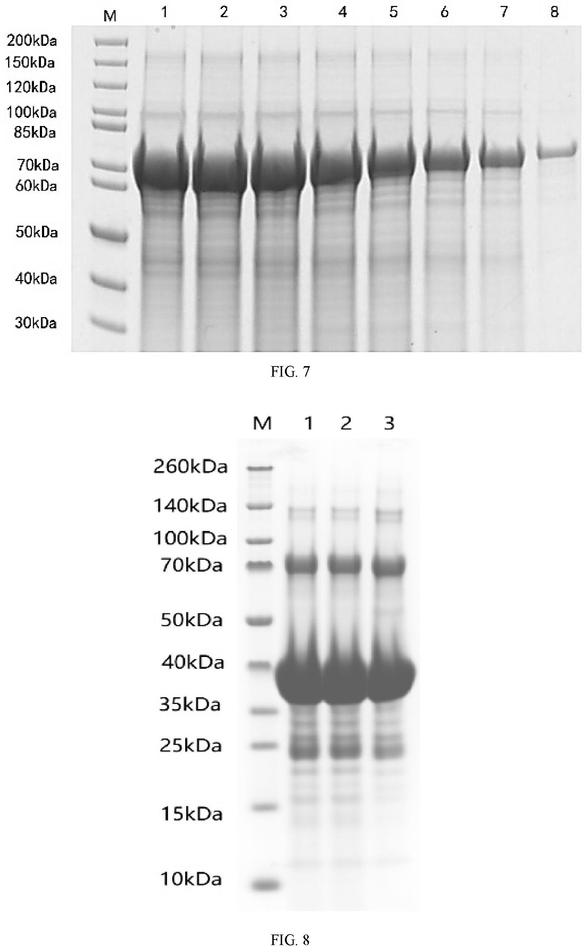

The electrophoresis results showed that the four proteins Mtb32a-4T, Ag85a-4T, ESAT6-CFP10-4T, and RV2660-TB10.4-4T were all obtained by purification, with purities of greater than 90%, as shown in FIGs. 3-6. The control protein M72-4T was also successfully renatured and obtained by purification, as shown in FIG. 7. In FIG. 3, M denotes the protein molecule marker, and lanes 1-6 denote Mtb32a-4T protein; in FIG. 4, M denotes the protein molecule marker, and lanes 1-10 denote Ag85a-4T protein; in FIG. 5, M denotes the protein molecule marker, and lanes 1-5 denote ESAT6-CFP10-4T protein; in FIG. 6, M denotes the protein molecule marker, and lanes 1-7 denote RV2660-TB10.4-4T protein.

### Example 4: Expression, purification, and preparation of M72 fusion protein

### 1) Treatment before purification

According to the procedures in the "Expression of recombinant protein" in Example 1, the M72-4T protein was expressed and the bacterial sludge was collected. The M72-4T expression bacterial sludge (corresponding to 200 mL of the expression bacterial solution) was resuspended in a 20 mL buffer containing 20 mM Tris-HCl, 150 mM NaCl, pH 7.4. and subjected to ultrasonic crushing. The cells were lysed by ultrasonication and centrifuged at 13,000 g/min for 30 min at 4 °C. The supernatant was discarded, and the pellet was retained, resuspended and dissolved in a buffer containing 20 mM Tris-HCl, 150 mM NaCl, 8 M Urea, pH 7.4, and centrifuged at 13,000 g/min and 4 °C for 30 min. The supernatant was retained.

### 2) Nickel ion affinity chromatography

Affinity purification was accomplished using a nickel ion affinity packing chromatography column. The volume of the chromatography column was 10 mL, the flow rate for chromatography was 5 mL/min, and 70 mL of sample was loaded. Chromatographic procedure: The Ni-Bestarose Fast Flow column was sterilized and equilibrated with a capture buffer containing 20 mM Tris-HCl, 150 mM NaCl, 8 M Urea, pH 7.4. The sample was loaded, and column was washed with a solution containing 20 mM Tris-HCl, 150 mM NaCl, 8 M Urea, pH 7.4, washed with a solution containing 20 mM Tris-HCl, 150 mM NaCl, 8 M Urea, 2% Triton-X100, pH 7.4 to remove endotoxins, washed with a buffer containing 20 mM imidazole + 20 mM Tris-HCl, 150 mM NaCl, 8 M Urea, pH 7.4 to remove protein impurities, and eluted with a buffer containing 500 mM imidazole + 220 mM Tris-HCl, 150 mM NaCl, 8 M Urea, pH 7.4 to harvest M72 antigenic component.

### 3) Ultrafiltration through membrane and renaturation

The M72-4T antigen purified by nickel ion affinity chromatography was concentrated by tangential flow ultrafiltration using a 10 kDa ultrafiltration membrane, and then a buffer containing 20 mM Tris-HCl, 150 mM NaCl, pH 7.4 was continuously added to the concentrated protein solution. Urea in the M72-4T antigen was gradually removed, and the M72 antigen was completely renatured in a buffer containing 20 mM Tris-HCl, 150 mM NaCl, pH 7.4.

### 4) Size-exclusion chromatographic purification

The renatured M72-4T antigen was purified using a HiLoad 16/600 Superdex 200 pg column. The column volume of the SEC column was 120 mL, and the loading amount of affinity-purified M72-4T sample was controlled at approximately 4% of the column volume.

Chromatographic procedure: The Superdex 200 pg was sterilized and equilibrated with an equilibration buffer (20 mM Tris-HCl, 150 mM NaCl, pH 7.4). The sample was loaded, and the column was washed with a solution containing 20 mM Tris-HCl, 150 mM NaCl, pH 7.4. The M72-4T antigenic component was collected. FIG. 7 shows the results of size-exclusion chromatographic separation and purification of M72-4T protein (M denotes the protein molecule marker, and lanes 1-8 denote M72-4T protein).

### Example 5: Construction, expression, and purification of coding gene of binding peptide 2-NPM fusion protein

### 1. Construction and expression

The sequence of the NPM-4C protein was subjected to *E. coli* expression host codon optimization, gene synthesis, and subcloning, and the coding gene of the fusion protein was constructed into pET30a for expression in *E. coli* BL21(DE3). The cells were harvested and then lysed by high-pressure homogenization to release the target protein, and the feed liquid was clarified to remove bacterial debris and protein impurities. The amino acid and nucleotide sequences of NPM and NPM-4C are shown in Tables 3 and 4.

### 2. Pretreatment before chromatography

The clarification of the feed liquid was mainly achieved by heating treatment. The heating treatment was performed using a two-step heating method. The *E. coli* lysate supernatant was subjected to first-step heating and second-step heating (i.e., "two-step heating"). The impurity removal effect and the purity of the recombinant particle protein component in the two-step heating process were measured.

60 g of wet *E. coli* cells collected by centrifugation were taken, resuspended in 240 mL of buffer (20 mM Tris-HCl, 2 mM PMSF, pH = 9.0), and lysed using a high-pressure homogenizer at 1000 bar. After centrifugation, 280 mL of supernatant was collected. 40 mL of the resulting supernatant was then taken and subjected to the two-step heating operation. The lysate supernatant, the supernatant obtained after the first-step heating and centrifugation, and the resuspension of the pellet obtained after the second-step heating and centrifugation were subjected to SDS-PAGE analysis.

As shown in Table 5, in the first-step heating treatment, the pH was adjusted to 9.0, and the sample was heated in a water bath at 80 °C for 1 h. After returning to room temperature, centrifugation was performed to collect a supernatant (about 35 mL). In the second-step heating treatment, 35 mL of a buffer containing 100 mM Tris-HCl, 5 mM EDTA, pH 7.4, and 4% Triton was added, and then 7 mL of 1 M Tris-HCl was added. The mixture was thoroughly mixed, heated in a water bath at 60 °C for 10 min, and then immediately centrifuged to collect a pellet. The pellet was then redissolved in a buffer containing 20 mM Tris-HCl, 5 mM EDTA, pH 9.0.

**Table 3: Amino acid sequences of NPM, NPM-4C, binding peptide 2, linker peptide 2, and fusion protein in examples of the present application**

| Polypeptide name | Amino acid sequence |
|---|---|
| Binding peptide 2 (4C) | MDSATHIKFSKRDEDGKELAGATMELRDSSGKTISTWISDGQVKDFYLYPGKYTFVETAAPDGYE VATAITFTVNEQGQVTVNGKATKGDAHI (SEQ ID NO:24) |
| Linker peptide 2 | GGSGGSGGSGGS (SEQ ID NO: 25) |
| NPM | MKMEELFKKHKIVAVLRANSVEEAKKKALAVFLGGVHLIEITFTVPDADTVIKELSFLKEMGAIIG AGTVTSVEQARKAVESGAEFIVSPHLDEEISQFAKEKGVFYMPGVMTPTELVKAMKLGHTILKLF PGEVVGPQFVKAMKGPFPNVKFVPTGGVNLDNVCEWFKAGVLAVGVGSALVKGTPVEVAEKA KAFVEKIRGCTE (SEQ ID NO:26) |
| NPM-linker peptide 2-binding peptide 2 (NPM-4C) | MDSATHIKFSKRDEDGKELAGATMELRDSSGKTISTWISDGQVKDFYLYPGKYTFVETAAPDGYE VATAITFTVNEQGQVTVNGKATKGDAHIGGSGGSGGSGGSMKMEELFKKHKIVAVLRANSVEEA KKKALAVFLGGVHLIEITFTVPDADTVIKELSFLKEMGAIIGAGTVTSVEQARKAVESGAEFIVSPH LDEEISQFAKEKGVFYMPGVMTPTELVKAMKLGHTILKLFPGEVVGPQFVKAMKGPFPNVKFVP TGGVNLDNVCEWFKAGVLAVGVGSALVKGTPVEVAEKAKAFVEKIRGCTE (SEQ ID NO:27) |

**Table 4: Nucleotide sequences of NPM, NPM-4C, binding peptide 2, linker peptide 2, and fusion protein in examples of the present application**

| Polypeptide name | Nucleotide sequence |
|---|---|
| Binding peptide 2 (4C) | ATGGACTCTGCTACTCACATCAAATTCTCTAAACGCGACGAAGACGGCAAAGAACTGGCAG GCGCTACTATGGAACTGCGTGACTCCAGCGGCAAAACTATCTCCACTTGGATCTCCGACGG TCAGGTGAAGGATTTCTACCTGTACCCGGGCAAATACACCTTCGTTGAAACTGCAGCACCT GACGGTTACGAAGTAGCAACTGCTATCACGTTCACCGTTAACGAGCAGGGTCAGGTTACCG TTAACGGCAAGGCGACTAAGGGTGATGCACACATC (SEQ ID NO:28) |
| Linker peptide 2 | GGTGGTTCTGGCGGCTCTGGTGGTTCTGGTGGCTCT (SEQ ID NO: 29) |
| NPM | ATGAAAATGGAAGAGCTGTTCAAAAAACACAAAATCGTTGCGGTACTGCGCGCAAATTCC GTCGAAGAGGCTAAAAAAAAAGCTCTGGCTGTTTTCCTGGGTGGCGTGCATCTGATCGAAA TCACCTTTACCGTACCTGATGCTGATACGGTGATCAAGGAACTGTCTTTCCTGAAGGAAATG GGCGCTATCATCGGTGCTGGCACCGTTACCAGCGTTGAACAGGCACGTAAGGCTGTTGAAA GCGGCGCGGAGTTCATCGTTTCTCCGCATCTGGATGAAGAAATCTCCCAGTTCGCGAAAGA AAAGGGTGTCTTCTATATGCCGGGCGTGATGACTCCAACTGAACTGGTCAAAGCGATGAAA CTGGGTCACACCATCCTGAAACTGTTTCCAGGTGAAGTGGTTGGTCCACAGTTCGTGAAAG CCATGAAAGGCCCGTTCCCTAACGTTAAATTCGTCCCGACCGGCGGTGTAAACCTGGATAA CGTTTGCGAATGGTTCAAAGCGGGTGTACTGGCTGTTGGTGTTGGTTCCGCACTGGTTAAAG GCACGCCAGTGGAAGTAGCTGAAAAAGCCAAAGCCTTCGTGGAAAAGATCCGTGGCTGTA CCGAA (SEQ ID NO:30) |
| NPM-4C | ATGGACTCTGCTACTCACATCAAATTCTCTAAACGCGACGAAGACGGCAAAGAACTGGCAG GCGCTACTATGGAACTGCGTGACTCCAGCGGCAAAACTATCTCCACTTGGATCTCCGACGG TCAGGTGAAGGATTTCTACCTGTACCCGGGCAAATACACCTTCGTTGAAACTGCAGCACCT GACGGTTACGAAGTAGCAACTGCTATCACGTTCACCGTTAACGAGCAGGGTCAGGTTACCG TTAACGGCAAGGCGACTAAGGGTGATGCACACATCGGTGGTTCTGGCGGCTCTGGTGGTTC TGGTGGCTCTATGAAAATGGAAGAGCTGTTCAAAAAACACAAAATCGTTGCGGTACTGCGC GCAAATTCCGTCGAAGAGGCTAAAAAAAAAGCTCTGGCTGTTTTCCTGGGTGGCGTGCATC TGATCGAAATCACCTTTACCGTACCTGATGCTGATACGGTGATCAAGGAACTGTCTTTCCTG AAGGAAATGGGCGCTATCATCGGTGCTGGCACCGTTACCAGCGTTGAACAGGCACGTAAG GCTGTTGAAAGCGGCGCGGAGTTCATCGTTTCTCCGCATCTGGATGAAGAAATCTCCCAGT TCGCGAAAGAAAAGGGTGTCTTCTATATGCCGGGCGTGATGACTCCAACTGAACTGGTCAA AGCGATGAAACTGGGTCACACCATCCTGAAACTGTTTCCAGGTGAAGTGGTTGGTCCACAG TTCGTGAAAGCCATGAAAGGCCCGTTCCCTAACGTTAAATTCGTCCCGACCGGCGGTGTAA ACCTGGATAACGTTTGCGAATGGTTCAAAGCGGGTGTACTGGCTGTTGGTGTTGGTTCCGC ACTGGTTAAAGGCACGCCAGTGGAAGTAGCTGAAAAAGCCAAAGCCTTCGTGGAAAAGAT CCGTGGCTGTACCGAA (SEQ ID NO:31) |

**Table 5: Pretreatment before chromatography**

| Procedures | Reaction condition | Operational value |
|---|---|---|
| First pelleting and centrifugation | Heating temperature | 80 °C |
| | Heating duration | 60 min |
| | Centrifugation rotation speed | 12000 g |
| | Centrifugation temperature | 4 °C |
| | Centrifugation duration | 30 min |
| Supernatant dilution | Dilution buffer | 100 mM Tris-HCl, 5 mM EDTA, 4% Triton, pH 7.4 |
| | Volume of dilution buffer | 1:1 (v/v) |
| | pH buffer | 1M Tris-HCl, pH 7.4 |
| | Volume of pH buffer | 10% of the total volume |
| Second pelleting and centrifugation | Heating temperature | 60 °C |
| | Heating duration | 10 min |
| | Centrifugation rotation speed | 6000 g |
| | Centrifugation temperature | 30 °C |
| | Centrifugation duration | 10 min |
| Pellet resuspending | Resuspension buffer | 20 mM Tris-HCl, 5 mM EDTA, pH 9.0 |
| | Volume of resuspension buffer | Resuspending to pre-centrifugation volume |
| | pH | 9.0+0.1 |
| | Filtration | 0.22 µm |

After the two-step heating process, adding urea and sodium chloride at different concentrations before chromatographic purification could significantly reduce the presence of unidentified substances near the target recombinant particle protein bands. The preferred pretreatment condition for the recombinant particle protein component before Fractogel DEAE M chromatography was soaking in 8 M urea and 50-200 mM sodium chloride.

### 3. Chromatographic purification

The above recombinant particle protein component sample solution was refined by ion exchange chromatography and hydrophobic chromatography. The first-step chromatographic purification was performed using the Fractogel DEAE M chromatography process for chromatographic purification. The specific procedures and parameters are shown in Table 6. The collected Fractogel DEAE M elution fraction sample was first diluted in a buffer, and then 50% (w/v) sucrose stabilizer was added to prevent pelleting of the recombinant particle protein component during the next chromatography step. The specific parameters are shown in Table 7. Then, the sample was further refined using a hydrophobic chromatography process with Octyl Bestarose 4FF (second-step chromatographic purification). The specific procedures and parameters are shown in Table 8.

Method for first-step chromatography: chromatography packing-Fractogel DEAE M, retention time-12.5 min.

**Table 6. Procedures for first-step chromatography**

| Chromatography procedures | Chromatography buffer/condition | Parameter |
|---|---|---|
| Equilibration buffer | 20 mM Tris-HCl, 5 mM EDTA, 8 M Urea, 50 mM NaCl, pH 9.0 | 6 CV |
| pH after equilibration | 8.8±0.05 | 8.80 |
| Rinse buffer 1 | 20 mM Tris-HCl, 5 mM EDTA, 8 M Urea, 50 mM NaCl, pH 9.0 | 1.5 CV |
| Rinse buffer 2 | 20 mM Tris-HCl, 5 mM EDTA, 8 M Urea, 2% Triton, pH 9.0 | 5 CV |
| Rinse buffer 3 | 20 mM Tris-HCl, 8 M Urea, pH 9.0 | 5 CV |
| Rinse buffer 4 | 20 mM Tris-HCl, 4 M Urea, pH 9.0 | 5 CV |
| Elution buffer | 20 mM Tris-HCl, 4 M Urea, 150 mM, pH 9.0 | 2 CV |
| Collection range | 50 mAU-50 mAU | Optical path length of 2 mm |

**Table 7. Procedures for sample dilution before second-step chromatography**

| Procedures | Buffer for dilution | Dilution volume |
|---|---|---|
| Collection of eluate from first-step chromatography | N/A | N/A |
| Buffer dilution | 20 mM Tris-HCl, 1 M NaCl, 50% (w/v) sucrose, pH 9.0 | 2× the volume of eluate |
| Buffer dilution | 20 mM Tris-HCl, 2 M NaCl, pH 9.0 | 1× the volume of eluate |

Method for second-step chromatography: chromatography packing-Octyl Bestarose 4FF, retention time-12.5 min

**Table 8. Procedures for second-step chromatography**

| Chromatography procedures | Chromatography buffer/condition | Parameter |
|---|---|---|
| Equilibration buffer | 20 mM Tris-HCl, 1 M NaCl, 25% (w/v) sucrose, pH 9.0 | 2 CV |
| Rinsing buffer | 20 mM Tris-HCl, 1 M NaCl, 25% (w/v) sucrose, pH 9.0 | 1.5 CV |
| Elution buffer | 20 mM Tris-HCl, 25% (w/v) sucrose, pH 9.0 | 3 CV |
| Collection range | 50 mAU-50 mAU | Optical path length of 2 mm |

### Results and analysis:

Through purity testing, it was found that after further refining using the above chromatography medium combination, the purity of the resulting product reached 99.0% or higher. The SDS-PAGE analysis results of the separated and purified NPM-4C protein by Octyl Bestarose 4FF are shown in FIG. 8 (M: protein molecule marker; lanes 1-2: NPM-4C protein).

### Example 6: Binding of M. tuberculosis structural protein to NPM, purification of binding product, and particle characterization

### 1. Binding of antigen to NPM

The high-purity Mtb32a-4T, Ag85a-4T, ESAT6-CFP10-4T, and RV2660-TB10.4-4T antigens obtained by size-exclusion chromatographic purification were mixed with NPM-4C at a BCA protein concentration ratio of 6:1. A 50% sucrose stock solution was added at a final sucrose concentration of about 25%, and a 1 M Tris-HCl stock solution with a total reaction volume of 10% was added to stabilize the pH. The binding reaction was carried out at 22 °C for 48 h. As an example, the Mtb32a-NPM binding system specifically may be: 6 mL of Mtb32a-4T (1 mg/mL), 1 mL of NPM-4C (1 mg/mL), 8.75 mL of 50% sucrose, and 1.75 mL of 1 M Tris-HCl 7.4 in a total volume of 17.5 mL.

### 2. Purification of binding products

The Mtb32a-NPM, Ag85a-NPM, ESAT6-CFP10-NPM, and RV2660-TB10.4-NPM binding products were purified using Cytiva HiLoad 16/600 Superdex 200pg (column volume 120 mL) or Cytiva Superdex 200 Increase 10/300 GL (column volume 23 mL), and the Mtb32a, Ag85a, ESAT6-CFP10, RV2660-TB10.4 antigens not bound to NPM-4C were isolated and removed. If the SEC column HiLoad 16/600 Superdex 200pg was used, the sample load amount of the Mtb32a-NPM, Ag85a-NPM, ESAT6-CFP10-NPM, and RV2660-TB10.4-NPM binding samples was controlled at about 3%-6%; if the SEC column Superdex 200 Increase 10/300 GL was used, the sample load amount of the Mtb32a-NPM, Ag85a-NPM, ESAT6-CFP10-NPM, and RV2660-TB10.4-NPM binding samples was controlled at 0.5-1 mL.

Chromatographic program: The Superdex 200pg or Superdex 200 Increase column was sterilized and equilibrated with an equilibration buffer containing 12.5% sucrose in TBS (20 mM Tris-HCl, 150 mM NaCl, 12.5% sucrose Mtb). The sample was loaded, and the column was washed with the TBS solution containing 12.5% sucrose. Mtb32a-NPM, Ag85a-NPM, ESAT6-CFP10-NPM, and RV2660-TB10.4-NPM components were collected and subjected to SDS-PAGE analysis according to the method for SDS-PAGE analysis of the purified antigens in Example 3.

### Results and analysis:

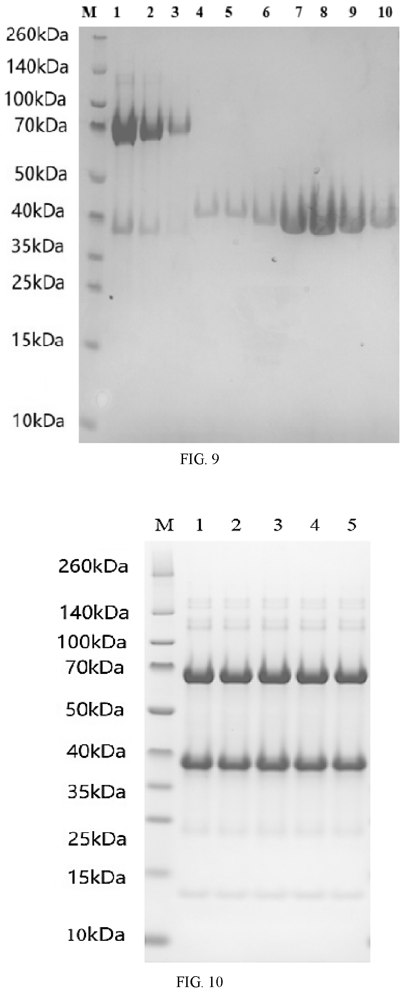

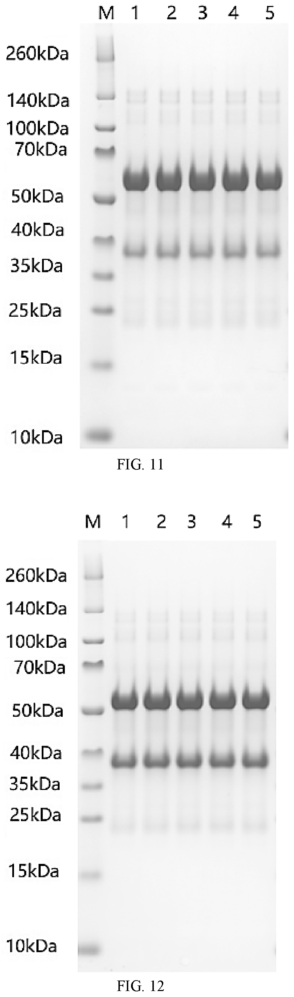

SDS-PAGE analysis was performed on Mtb32a-NPM, Ag85a-NPM, ESAT6-CFP10-NPM, and RV2660-TB10.4-NPM particles purified by size-exclusion chromatography, and the results show that the purity could reach 90% or higher. The specific results are shown in FIGs. 9-12. In FIG. 9, M denotes the protein molecule marker, lanes 1-3 denote Mtb32a-NPM binding products, and lanes 4-10 denote unbound Mtb32a; in FIG. 10, M denotes the protein molecule marker, and lanes 1-5 denote Ag85a-NPM binding products; in FIG. 11, M denotes the protein molecule marker, and lanes 1-5 denote ESAT6-CFP10-NPM binding products; in FIG. 12, M denotes the protein molecule marker, and lanes 1-5 denote RV2660-TB10.4-NPM binding products.

Mtb32a, Ag85a, ESAT6-CFP10, and RV2660-TB10.4 were subjected to a binding reaction with NPM-4C according to the method described above, and the binding rate was 82.5% as measured by SDS-PAGE grayscale method.

### 3. Particle characterization

### 1) TEM examination

Samples were prepared by negatively staining the Mtb32a-NPM, Ag85a-NPM, ESAT6-CFP10-NPM, and RV2660-TB10.4-NPM particles prepared in the above "Purification of binding products" using the floating method. A 400-mesh grid with a support film was selected and pre-treated to render it hydrophilic. Deionized water and a 2% uranyl formate negative staining solution were prepared. 3 µL of the prepared protein sample (0.1 mg/mL) was directly dropwise added to one side of the grid where the support film was located. After 1 minute, excess liquid was removed by touching the edge of the grid with clean filter paper. The grid was then briefly air-dried before being sequentially and rapidly rinsed twice on droplets of deionized water. This was followed by a single rinse with 5 µL of negative staining solution. Finally, 5 µL of negative staining solution was dripped to the grid and the grid was allowed to stand for 1 min. Then, the grid was held with tweezers, and the staining solution was removed using filter paper, leaving a thin layer to air-dry naturally before examination. The grid was examined under a 120 kV transmission electron microscope (FERRITINI Tecnai Spirit). The overall staining of the grid was assessed at low magnification. Holes with suitable thickness were selected for observation, and appropriate areas were chosen for photographing and data collection at high magnification.

### 2) DLS detection

The purified and prepared Mtb32a-NPM, Ag85a-NPM, ESAT6-CFP10-NPM, and RV2660-TB10.4-NPM particles were diluted to 0.15 mg/mL. A Zetasizer Lab instrument was used, ≥ 1 mL of the test sample was injected into a sample cell, and the instrument was run for measurement. Data analysis was performed based on the Z-average (nm), polydispersity index (PI) value, and the distribution profiles of the Size Distribution by Intensity/Volume. The results were then documented.

### Results and analysis:

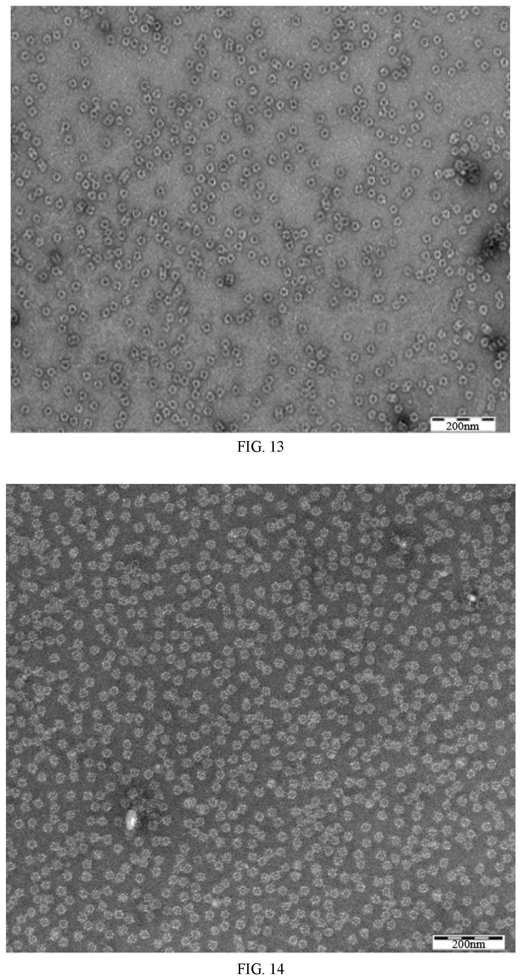

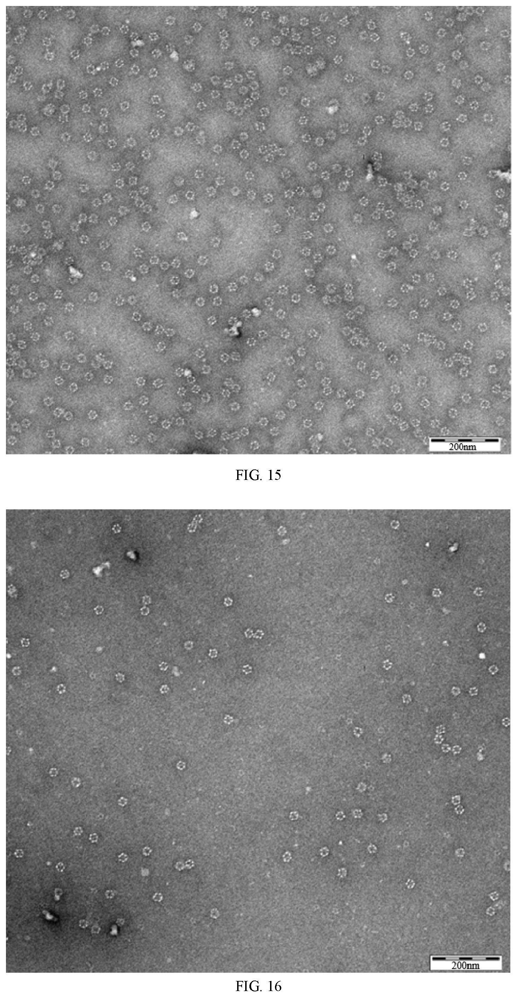

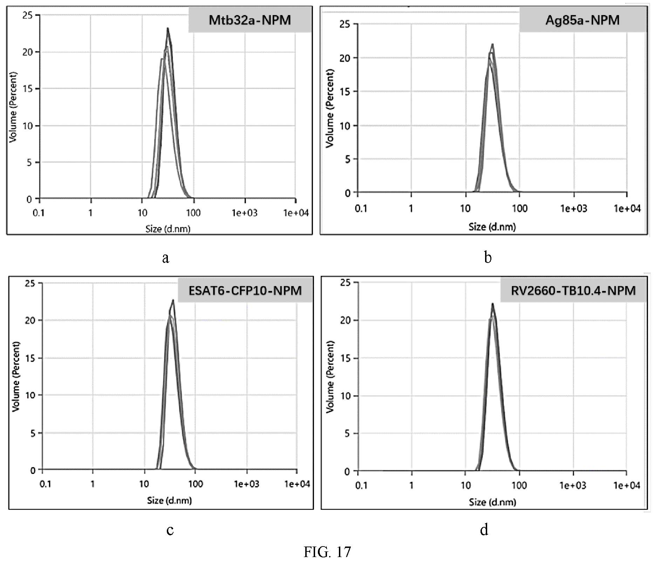

The photographs of electron microscope detection results of particles of Mtb32a-NPM, Ag85a-NPM, ESAT6-CFP10-NPM, and RV2660-TB10.4-NPM (0.1 mg/mL, 18,500×) show that the particles were uniformly distributed without aggregation, as shown in FIGs. 13-16. The distribution curve by intensity/volume analyzed by Zetasizer Lab instrument is shown in FIG. 17, and the results show that the peaks of the prepared Mtb32a-NPM, Ag85a-NPM, ESAT6-CFP10-NPM, and RV2660-TB10.4-NPM particles exhibited good overlap and uniform particle size distribution. The DLS results show that the particle sizes of Mtb32a-NPM, Ag85a-NPM, ESAT6-CFP10-NPM, and RV2660-TB10.4-NPM were 37.0 nm, 38.7 nm, 43.0 nm, and 39.8 nm, respectively, as shown in Table 9.

**Table 9. Z-average (nm) and polydispersity index (PI) values of prepared particulate antigens**

| Sample name | Z-Average (nm) | Polydispersity (PI) |
|---|---|---|
| Mtb32a-NPM | 37.0 | 0.17 |
| Ag85a -NPM | 38.7 | 0.13 |
| ESAT6-CFP10-NPM | 43.0 | 0.14 |
| RV2660-TB10.4-NPM | 39.8 | 0.14 |

### Example 7: Preparation of vaccines

### 1. Antigen and adjuvant of test vaccines

### 1) Drug substance of test vaccines

The drug substance of the test vaccine proteins were prepared by Guangzhou Patronus Biotech Co., Ltd., including Mtb32a-NPM, Ag85a-NPM, ESAT6-CFP10-NPM, and RV2660-TB10.4-NPM particles, Mtb32a-4T, Ag85a-4T, ESAT6-CFP10-4T, and RV2660-TB10.4-4T antigens, and M72-4T control antigen.

### 2) Adjuvant of test vaccines

On the basis of the commercial AS01B adjuvant, QS21 (5 µg) was added to small unilamellar vesicles (SUVs) of dioleoyl phosphatidylcholine (100 µg) containing cholesterol (25 µg) to prepare dual-strength AS01B (WO 96/33739) and monophosphoryl lipid A (MPL) (5 µg) in the membrane.

### 2. Preparation of test vaccines

### 1) Mtb recombinant antigen mix vaccine

Aliquots (50 µL) for injection were prepared by mixing 0.8 µg of buffer (TBS Mtb) of the protein mixture (0.2 µg each of Mtb32a-4T, Ag85a-4T, ESAT6-CFP10-4T, and RV2660-TB10.4-4T) with 50 µL of dual-strength AS01B.

### 2) Mtb nanoparticle antigen mix vaccine

Aliquots (50 µL) for injection were prepared by mixing 0.8 µg of buffer (TBS Mtb) of the protein mixture (0.2 µg each of Mtb32a-NPM, Ag85a-NPM, ESAT6-CFP10-NPM, and RV2660-TB10.4-NPM) with 50 µL of dual-strength AS01B.

### 3) Single Mtb nanoparticle antigen vaccine

Aliquots (50 µL) for injection were prepared by mixing 0.8 µg of buffer (TBS Mtb) of the Mtb32a-NPM, Ag85a-NPM, ESAT6-CFP10-NPM, and RV2660-TB10.4-NPM protein mixture with 50 µL of dual-strength AS01B.

### 4) M72 control vaccine

Aliquots (50 µL) for injection were prepared by mixing 0.8 µg of buffer (TBS Mtb) of the M72-4T protein mixture with 50 µL of dual-strength AS01B.

### Example 8: Vaccine immune protection study

### 1. Experimental animals and grouping

Female C57BL/6 mice aged 4-6 weeks, female, purchased from Vital River were selected. The mice passing the quarantine were marked with metal ear tags, randomly grouped by body weight, housed after grouping, and given free access to food and water. 6 vaccine candidate immunization groups (12 mice per group), an M72-4T group (12 mice), a BCG control group (12 mice), and a normal saline group (15 mice) were set. The animals were housed in an SPF animal room and provided with sterile feed and sterilized deionized water specialized for SPF animals. The housing environment was maintained on a 12-h light/dark cycle, at a temperature of 21±2 °C and a humidity of 30-70%.

### 2. Immunization and challenge

The vaccine groups (vac-1 to vac-6) and the M72-4T group (Control-A) were immunized twice at a dose of 100 µL (0.8 µg) with an interval of 3 weeks. The normal saline group was immunized with the same volume of normal saline. The mice in the BCG group (Control-B) were immunized subcutaneously with BCG cells once at 5 × 10⁴ CFU/100 µL/mouse. The remaining groups were immunized by intramuscular injection.

Four weeks after the last immunization, the mice were challenged with an aerosol at a challenge dose of 100 cells/mouse. Four weeks after the challenge, the mice were dissected. Pathological sections of the lungs and spleen were prepared, and the grinding solutions of the lungs and spleen were used to calculate the bacterial load. The specific grouping scheme, immunization schedule, and challenge schedule are shown in Tables 10 and 11:

**Table 10: Immunization and challenge schedule**

| Group No. | C57BL/ (4-6 weeks) | Immunization route | Immunization Doses | Interval/weeks | Challenge/weeks | Anatomy/ weeks | Culture assay/ weeks |
|---|---|---|---|---|---|---|---|
| vac-1 | 12 mice | Intramuscular injection | 2 | 3 | 7 | 11 | 15 |
| vac-2 | 12 mice | | 2 | 3 | 7 | 11 | 15 |
| vac-3 | 12 mice | | 2 | 3 | 7 | 11 | 15 |
| vac-4 | 12 mice | | 2 | 3 | 7 | 11 | 15 |
| vac-5 | 12 mice | | 2 | 3 | 7 | 11 | 15 |
| vac-6 | 12 mice | | 2 | 3 | 7 | 11 | 15 |
| Control-A | 12 mice | | 2 | 3 | 7 | 11 | 15 |
| Control-B | 12 mice | Subcutaneous injection | 1 | - | 7 | 11 | 15 |
| Normal saline | 15 mice | Intramuscular injection | 2 | 3 | 7 | 11 | 15 |

**Table 11. Grouping and protein component, dose, and adjuvant**

| Group No. | Antigen | Adjuvant | Dose |
|---|---|---|---|
| vac-1 | 014-NPM, 072-NPM, 076-NPM, 077-NPM | AS01B | 0.2 µg×4=0.8 µg |
| vac-2 | 014, 072, 076, 077 | | 0.2 µg×4=0.8 µg |
| vac-3 | 072-NPM | | 0.8 µg |
| vac-4 | 076-NPM | | 0.8 µg |
| vac-5 | 077-NPM | | 0.8 µg |
| vac-6 | 014-NPM | | 0.8 µg |
| Control-A | M72-4T | | 0.8 µg |
| Control-B | BCG | - | 5×10⁴ CFU |
| Normal saline | - | - | - |

### 3. Detection of specific IgG after immunization

On D20 after the first immunization, the whole blood was collected into the centrifuge tube and left to stand at room temperature for 2 h or left to stand overnight in a refrigerator at 4 °C. After the blood was coagulated and the blood clot contracted, the blood was centrifuged at 4000 rpm for 10 min, and the supernatant was collected into a clean centrifuge tube and stored at -20 °C.

96-well microplates (Thermo Fisher Scientific) were separately coated with 014, 072, 076, and 077 proteins (at a concentration of 1 µg/mL) at 100 ng/50 µL/well and incubated at 4 °C overnight, and then the plate was washed twice with PBST (0.05% Tween 20). A blocking solution (Thermo Fisher Scientific) was added at 200 µL/well, followed by blocking at room temperature (25 °C±3 °C) for 1-4 h. Then, the plate was washed twice, diluted immune serum was added, and the mixture was incubated at room temperature for 1 h and then washed 4 times. HRP-IgG1 or HRP-IgG2a working solution diluted at 1:5000 was added at 50 µL/well. The plate was incubated at room temperature for 1 h and then washed 6 times, and a chromogenic solution was added at 100 µL/well. Color development was performed at room temperature for 10 min in the absence of light, and then 1 M HCl was added at 100 µL/well to stop the reaction. The main wavelength was set at 450 nm and the reference wavelength was set at 620 nm in the microplate reader. The absorbance value of the sample = OD450 - OD620. The measurement was completed within 5 min after the termination, and the humoral and cellular immune types were analyzed according to the detection results.

### Data processing:

The data were reliable when the following conditions were met: The OD value of the control serum ± 0.2, the OD value corresponding to the initial concentration of the sample was < 3.0, the OD value corresponding to the blank well was less than 0.1, and the coefficient of variation of duplicate wells (response values) should be less than 20%. The original sample data was applied to "excel Endpoint ELISA template" to determine the antibody titer. The results were analyzed using Graphpad Prism 9.1.2 software. Differences were analyzed using Unpaired t test or One-Way ANOVA. Two groups of data were defined as significantly different when P < 0.05.

The test results are as follows:

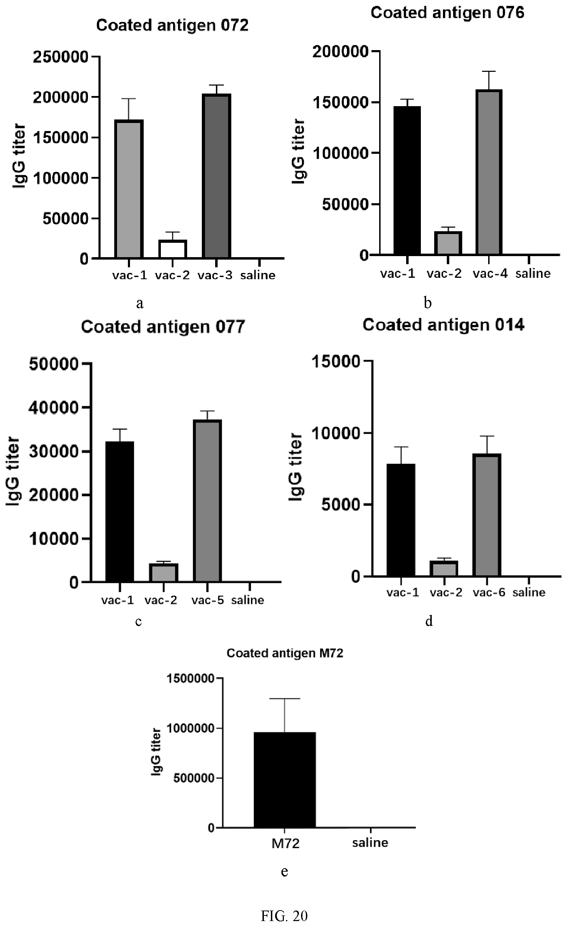

FIGs. 20a-d show the corresponding total IgG antibody levels in immunization serum of the different groups (vac-1, vac-2, single Mtb nanoparticle antigen vaccines, and normal saline) detected by using the 072, 076, 077, and 014 antigens, respectively, as the coating antigen, and FIG. 20e shows the IgG antibody levels detected by using M72 antigen (0.8 µg immunization group).

The results show that the single Mtb nanoparticle antigen vaccines (vac-3/4/5/6), the Mtb recombinant antigen mix vaccine (vac-2), and the Mtb nanoparticle antigen mix vaccine (vac-1) of the present disclosure all induced the production of IgG, indicating that all the vaccines of the present disclosure have good immunogenicity. The IgG antibody levels corresponding to vac-1 and the single Mtb nanoparticle antigen vaccine groups were all much higher than that of vac-2, indicating that the nanoparticle vaccines of the present disclosure can significantly improve the immunogenicity of the Mtb antigen.

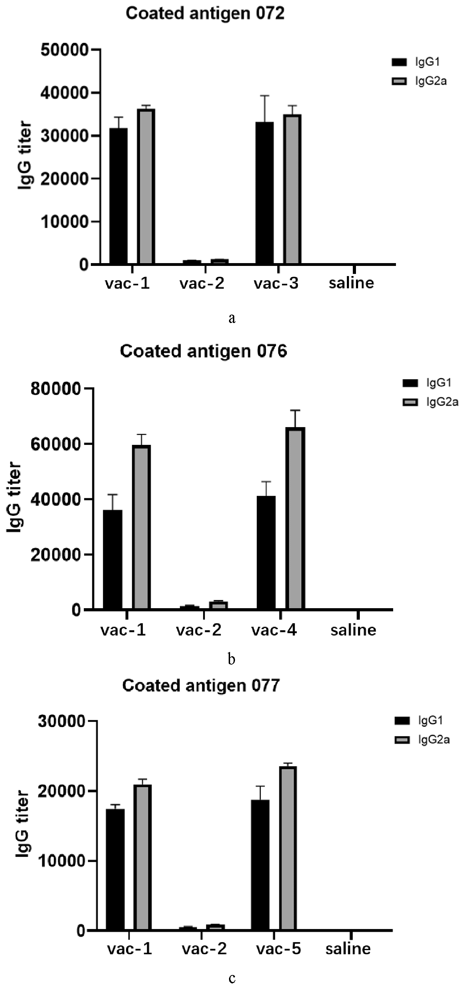

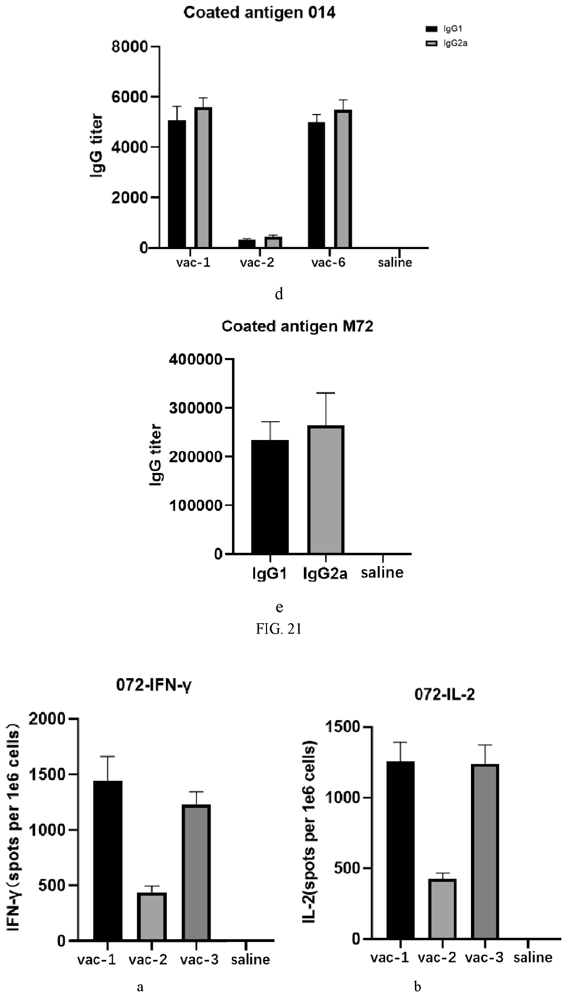

FIGs. 21a-d show the corresponding IgG1 and IgG2a antibody levels in the different groups (vac-1, vac-2, single Mtb nanoparticle antigen vaccines, and normal saline) detected by using the 072, 076, 077, and 014 antigens, respectively, as the coating antigen, and FIG. 21e shows the corresponding IgG1 and IgG2a antibody levels detected of the M72 group (0.8 µg immunization group).

The results showed that the IgG1 and IgG2a levels of the vac-1 and single Mtb nanoparticle antigen vaccine groups were much higher than those of the vac-2, and the IgG2a/IgG1 ratios of the vac-1 and single Mtb nanoparticle antigen vaccine groups were all greater than 1.0, indicating that 072, 076, 077, and 014 can all induce Th1-type cellular immune responses; the IgG2a/IgG1 ratio of the M72 group was greater than 1.0, indicating that it can induce Th1-type cellular immune responses, which is in line with literature reports, demonstrating that the control vaccine was successfully prepared.

### 4. Cytokine ELISA

1) Three weeks after the last immunization, 6 mice in each group were sacrificed. The spleen was collected, and splenocytes were isolated and seeded into a 96-well culture plate at 2.5 × 10⁵ cells/well.
2) The cells were cultured in a culture medium containing 014 (10 µg/mL), 072 (10 µg/mL), 076 (10 µg/mL), 077 (10 µg/mL), M72-4T (10 µg/mL), PPD (10 µg/mL), and Con A (3 µg/mL) in an incubator at 37 °C/5% CO₂ for 72 h.
3) The supernatant was collected and assayed for IFN-γ, TNFα, IL-4, and IL-2 by double antibody sandwich ELISA using a commercial kit; the results were analyzed using Graphpad Prism 9.1.2 software. Differences were analyzed using Unpaired t test or One-Way ANOVA. Two groups of data were defined as significantly different when P < 0.05.

### 5. Cytokine ELISPOT

1) A commercial 96-well filter plate was coated with IFN-γ, TNFα, IL-4, and IL-2 monoclonal antibodies and blocked. Three weeks after the last immunization, 6 mice in each group were sacrificed. The spleen was collected, and splenocytes were isolated and seeded into a 96-well culture plate at 2.0 × 10⁵ cells/well.
2) The cells were cultured in an unmodified culture medium or a culture medium containing 014 (10 µg/mL), 072 (10 µg/mL), 076 (10 µg/mL), 077 (10 µg/mL), M72-4T (10 µg/mL), PPD (10 µg/mL), and Con A (3 µg/mL) in an incubator at 37 °C/5% CO₂ for 48 h.
3) The wells of the plate were washed with PBS, and biotinylated mouse IFN-γ, TNFα, IL-4, and IL-2 secondary antibodies were added. The plate was incubated at room temperature for 2 h, and the filter membrane was subjected to a chromogenic reaction with a substrate according to the instructions of a commercial kit.
4) After the plate was dried, spots were counted and analyzed using an automated ELISPOT plate reader.

The results were analyzed using Graphpad Prism 9.1.2 software. Differences were analyzed using Unpaired t test or One-Way ANOVA. Two groups of data were defined as significantly different when P < 0.05.

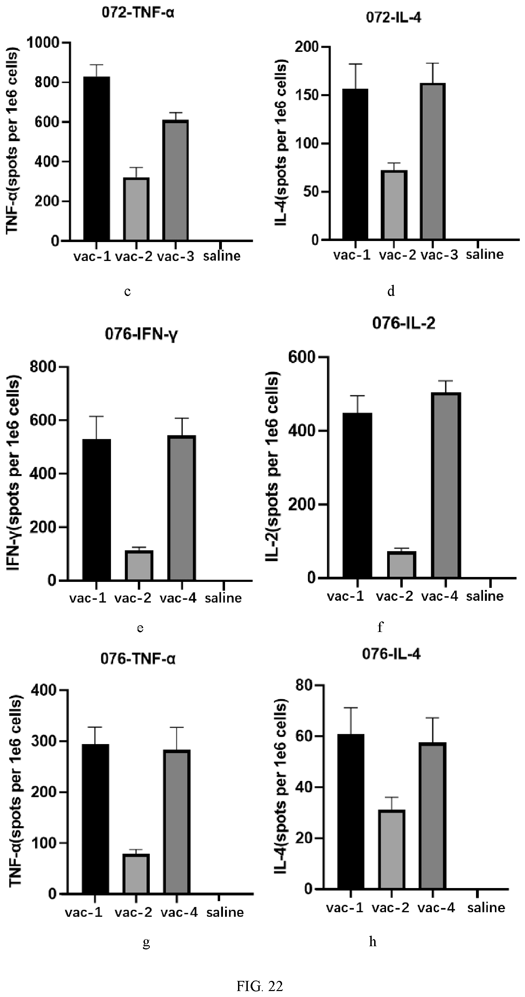

FIGs. 22a-d show the levels of IFN-γ, IL-2, TNFα, and IL-4, respectively, corresponding to different groups (vac-1, vac-2, single Mtb nanoparticle antigen vaccines, and normal saline) using 072 antigen as the specific antigen stimulator; FIGs. 22e-h show the levels of IFN-γ, IL-2, TNFα, and IL-4, respectively, corresponding to different groups (vac-1, vac-2, single Mtb nanoparticle antigen vaccines, and normal saline) using 076 antigen as the specific antigen stimulator.

The results show that the corresponding IFN-γ, IL-2, TNFα, and IL-4 levels in the vac-1 and single Mtb nanoparticle antigen vaccine groups stimulated by 072 and 076 were higher than those of vac-2, and the IFN-γ, IL-2, and TNFα levels in all group were significantly higher than that of IL-4, indicating that both 072 and 076 can cause cellular immune responses dominated by the Th1 type.

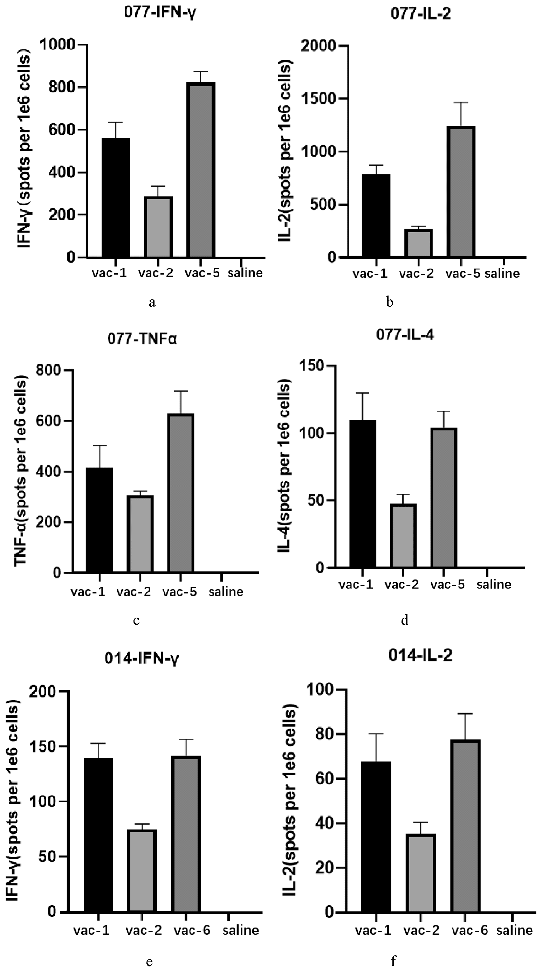

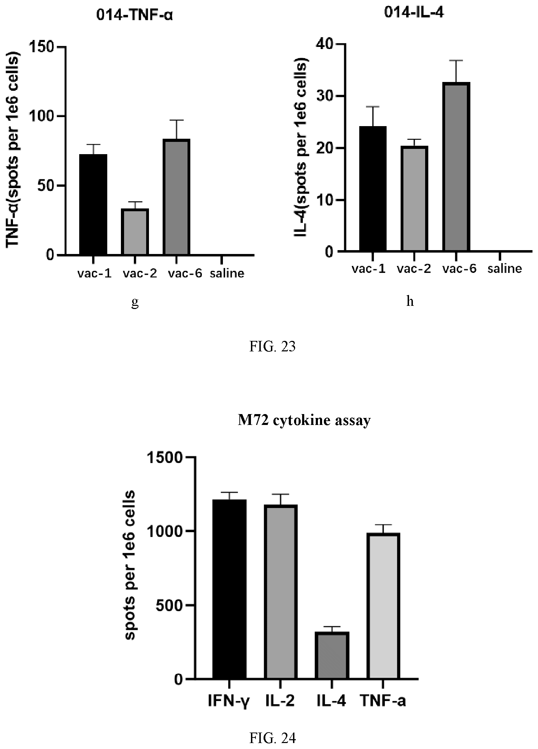

FIGs. 23 a-d show the levels of IFN-γ, IL-2, TNFα, and IL-4, respectively, corresponding to different groups (vac-1, vac-2, single Mtb nanoparticle antigen vaccines, and normal saline) using 077 antigen as the specific antigen stimulator; FIGs. 23e-h show the levels of IFN-γ, IL-2, TNFα, and IL-4, respectively, corresponding to different groups (vac-1, vac-2, single Mtb nanoparticle antigen vaccines, and normal saline) using 014 antigen as the specific antigen stimulator.

The results show that the corresponding IFN-γ, IL-2, TNFα, and IL-4 levels in the vac-1 and single Mtb nanoparticle antigen vaccine groups stimulated by 077 and 014 were higher than those of vac-2, and the IFN-γ, IL-2, and TNFα levels in all group were significantly higher than that of IL-4, indicating that both 077 and 014 can cause cellular immune responses dominated by the Th1 type.

FIG. 24 shows that the levels of IFN-γ, IL-2, and TNFα corresponding to the M72 group (0.8 µg immunization group) were significantly higher than that of IL-4, indicating that the prepared M72 vaccine can induce Th1-type cellular immune responses, which is in line with literature reports, demonstrating that the control vaccine was successfully prepared.

### 6. Flow cytometry assay of T-cell immune response

1) Flow cytometry detection of CD4+ and CD8+ T cells: 3 weeks after the last immunization, 6 mice in each group were sacrificed. The spleen was collected, and splenocytes (1 × 10⁶ cells) were isolated and harvested. The cells were washed with a staining buffer and stained with a mixture containing PE-Cyanine7 CD3 monoclonal antibody (1:50 final dilution, eBioscience), PE rat anti-mouse CD4 (1:50 final dilution, BD) and PerCP-Cy^{™}5.5 rat anti-mouse CD8 (1:50 final dilution, BD) in a total amount of 50 µL for 15 min. The cells were washed twice with 1× Perm/Wash solution and resuspended in 1× Perm/Wash solution, and then analyzed using DxFLEX (BECKMAN COULTER). The data were analyzed using CytExpert.
2) CD4+ and CD8+ T cells with specific IFN-γ, TNFα, IL-4, and IL-2 expression were analyzed using ICS and flow cytometry. Splenocytes cells (1 × 10⁶ cells) were isolated, harvested, and restimulated *in vitro* with 014 (10 µg/mL), 072 (10 µg/mL), 076 (10 µg/mL), 077 (10 µg/mL), M72-4T (10 µg/mL), and CD28/CD49d co-stimulatory antibodies (BD) for 6 hours. For intracellular cytokine staining, cells were co-incubated with a protein transport inhibitor (with Brefeldin A; BD) for 4h; cells were washed with PBS and stained with Fixable Viability Stain 780 (1:1000 final dilution, BD) and mouse Fc block (BD) for 15 min;
3) The cells were washed with a staining buffer and stained with a mixture containing PE-Cyanine7 CD3 monoclonal antibody (1:50 final dilution, eBioscience), PE rat anti-mouse CD4 (1:50 final dilution, BD) and PerCP-Cynce rat anti-mouse CD8 (1:50 final dilution, BD) in a total amount of 50 µL for 15 min. The cells were fixed and permeabilized with Fix/Perm solution kit (BD). After washing twice with 1× Perm/Wash solution and staining with APC rat anti-mouse IFN-y (1:50 final dilution, BD), FITC rat anti-mouse IL-2 (1:50 final dilution, BD), PE rat anti-mouse IL-4 (1:50 final dilution, BD), and R718 rat anti-mouse TNFα (1:50 final dilution, BD), the cells were washed with 1× Perm/Wash solution and resuspended, and then analyzed using DxFLEX (BECKMAN COULTER). Data were analyzed using CytExpert and expressed as the percentage of total frequency of CD4+ T/CD8+ T cells expressing IFN-γ, TNFα, IL-4, and IL-2, with background subtracted from the mean response of specific CD4+ and CD8+ T cells.

### 7. Statistics of challenge protection results

Four weeks after the last immunization, challenge was performed by exposure to a low-dose *M. tuberculosis* H37Rv strain aerosol, and the UW-madison aerosol contact chamber used was calibrated to deliver 50-100 CFU to the lungs. After 4 weeks, the mice were euthanized, and lung and spleen homogenates were prepared by grinding with PBS/Tween-80 (0.05%). The homogenates of individual intact organs were serially diluted and inoculated onto Middlebrook 7H11 Bacto agar medium, and bacterial colonies were calculated after incubation at 37 °C for 2-4 weeks in a humidified, 5% CO₂ condition. Final data are expressed as mean Log10 + SD of bacteria, reduction (difference) in Log10 of CFU = Log10 CFU of saline treatment - Log10 CFU of vaccine treatment.

### 8. Analysis of pathological sections

Four weeks after the challenge, the mice in all groups were euthanized, and spleen and lung tissues were collected. The tissues were fixed using a formaldehyde solution and sent to the company for HA staining. Pathological sections were prepared and analyzed.

### 9. Immune evaluation results

The detection results of specific IgG after immunization show that the antigens in the vac-1 to vac-6 groups can all well produce antibodies, and the single antigen groups and the mix antigen groups exhibited good immunogenicity; the cytokine ELISPOT results show that the antigens included in the vac-1 to vac-6 groups can all stimulate lymphocytes to produce high levels of IFN-γ, TNFα, and IL-2, and the screened antigens have a strong function of stimulating cellular immunity improvement.

Since *M. tuberculosis* mainly infects human macrophages and is an intracellular parasitic bacterium, the prevention and control of tuberculosis require intracellular anti-infection interventions. After the vaccine displays *M. tuberculosis* antigens 072, 076, 077, and 014 by means of NPM nanoparticles, it can effectively stimulate the body to produce high levels of IFN-γ, TNFα, and IL-2. Among them, IFN-γ can activate macrophages to kill *M. tuberculosis* and enhance the killing effect of NK cells, while IL-2 can also enhance the killing effect of NK cells. TNFα, as an important cytokine, plays an important role in the process of resisting the infection of *M. tuberculosis,* and its main function is to promote the apoptosis of infected macrophages and expose the hidden Mtb, such that the antigens are presented by APC to activate CTL immune generation.

It is found in the present application that the IgG levels of the prepared mix NPM group (vac-1) and the prepared single NPM groups of 072, 076, 077, and 014 were higher than that of the mix recombinant protein group (vac-2), and the IFN-γ, TNFα, IL-4, and IL-2 levels of the mix NPM group and the single NPM groups were also higher than those of the mix recombinant protein group, indicating that the *M. tuberculosis* nanoparticle antigens displayed by NPMs can better cooperate with adjuvants to improve the cellular immunity and humoral immunity levels, suggesting the advantages of the NPM nanoparticles in presenting the *M. tuberculosis* antigens. Also, the vaccines of the present disclosure can generate a significant Th1 immune response. The TB vaccines mainly depend on the cellular immune response, and the vaccine type biased towards the Th1 T cell immune response is in line with the immune preference of TB vaccine development.

The above results show that the antigens in the form of VLPs can improve the immune protection, and the nanoparticle antigens included in vac-1 to vac-6 can effectively stimulate the body to generate immune protection and effectively prevent the infection of *M. tuberculosis* H37Rv strain in mice, thus possessing great potential for development into TB subunit vaccines. The above recombinant nanoparticle *M. tuberculosis* subunit vaccines have great application prospects.

In summary, the above examples and drawings are only for the purpose of illustrating preferred examples disclosed herein, and are not intended to limit the protection scope of the present disclosure. Any modifications, equivalent substitutions, improvements, and the like made without departing from the spirit and principle of the present disclosure shall all fall within the protection scope of the present disclosure.

## Claims

1. An immunogenic complex, comprising:
(1) an antigenic component, comprising a *Mycobacterium tuberculosis* structural protein or an immunogenic fragment thereof and a binding peptide 1, the *M. tuberculosis* structural protein or the immunogenic fragment thereof and the binding peptide 1 forming a fusion protein; and
(2) a particle protein component, comprising a nanoparticle protein and a binding peptide 2, the nanoparticle protein and the binding peptide 2 forming a fusion protein,
wherein the *M. tuberculosis* structural protein or the immunogenic fragment thereof is selected from Mtb32a, Ag85a, ESAT6, CFP10, RV2660, or TB10.4, or a fusion protein formed by two or more of the structural proteins or immunogenic fragments thereof;
wherein the antigenic component and the particle protein component are covalently bound to each other via the binding peptide 1 and the binding peptide 2 to form the immunogenic complex.

2. The immunogenic complex according to claim 1, wherein the binding peptide 1 comprises the amino acid sequence as set forth in SEQ ID NO: 1, and the binding peptide 2 comprises the amino acid sequence as set forth in SEQ ID NO: 24;
preferably, the antigenic component further comprises a linker peptide 1, and the particle protein component further comprises a linker peptide 2; the antigenic component is formed by fusing the *M. tuberculosis* structural protein or an immunogenic fragment thereof, at the C-terminus, with the binding peptide 1 via the linker peptide 1; the particle protein component is formed by fusing the nanoparticle protein, at the N-terminus, with the binding peptide 2 via the linker peptide 2;
optionally, the linker peptide 1 is selected from the amino acid sequences of (GSG)ₙ, (GGGGS)ₙ, or (EAAAK)ₙ, wherein n may be an integer greater than 0 and less than or equal to 5; the linker peptide 2 is selected from the amino acid sequences of (GGS)ₙ, (SGGSGG)ₙ, or (GSGGSGGSG)ₙ, wherein n may be an integer greater than 0 and less than or equal to 10;
preferably, the linker peptide 1 comprises the amino acid sequence as set forth in SEQ ID NO: 2, and the linker peptide 2 comprises the amino acid sequence as set forth in SEQ ID NO: 25;
optionally, both the antigenic component and the particle protein component comprise a histidine tag.

3. The immunogenic complex according to claim 1 or 2, wherein the *M. tuberculosis* structural protein or the immunogenic fragment thereof or the fusion protein formed by the structural protein or the immunogenic fragment is selected from the following sequences:
(1) Mtb32a, comprising a sequence having 40% or higher, 50% or higher, 60% or higher, 70% or higher, 80% or higher, or 90% or higher identity to SEQ ID NO: 3;
(2) Ag85a, comprising a sequence having 40% or higher, 50% or higher, 60% or higher, 70% or higher, 80% or higher, or 90% or higher identity to SEQ ID NO: 5;
(3) ESAT6-CFP10, comprising a sequence having 40% or higher, 50% or higher, 60% or higher, 70% or higher, 80% or higher, or 90% or higher identity to SEQ ID NO: 7; or
(4) RV2660-TB10.4, comprising a sequence having 40% or higher, 50% or higher, 60% or higher, 70% or higher, 80% or higher, or 90% or higher identity to SEQ ID NO: 9;
preferably, the *M. tuberculosis* structural protein or the immunogenic fragment thereof or the fusion protein formed by the structural protein or the immunogenic fragment is selected from: Mtb32a set forth in SEQ ID NO: 3, Ag85a set forth in SEQ ID NO: 5, ESAT6-CFP10 set forth in SEQ ID NO: 7, or RV2660-TB10.4 set forth in SEQ ID NO: 9.

4. The immunogenic complex according to any one of claims 1-3, wherein the nanoparticle protein is NPM, AP205, or ferritin protein; the amino acid sequence of NPM is set forth in SEQ ID NO: 26.

5. The immunogenic complex according to any one of claims 1-4, wherein the immunogenic complex is **characterized by** any one or more of (1)-(8) below:
(1) the amino acid sequence of the *M. tuberculosis* structural protein or the immunogenic fragment thereof is set forth in SEQ ID NO: 3, SEQ ID NO: 5, SEQ ID NO: 7, or SEQ ID NO: 9;
(2) the amino acid sequence of the binding peptide 1 is set forth in SEQ ID NO: 1;
(3) the amino acid sequence of the linker peptide 1 is set forth in SEQ ID NO: 2;
(4) the nanoparticle protein is selected from NPM, AP205, or ferritin, wherein the amino acid sequence of NPM is set forth in SEQ ID NO: 26;
(5) the amino acid sequence of the binding peptide 2 is set forth in SEQ ID NO: 24;
(6) the amino acid sequence of the linker peptide 2 is set forth in SEQ ID NO: 25;
(7) the amino acid sequence of the antigenic component is selected from SEQ ID NO: 4, SEQ ID NO: 6, SEQ ID NO: 8, or SEQ ID NO: 10; and
(8) the amino acid sequence of the particle protein component is set forth in SEQ ID NO: 27.

6. A recombinant antigen protein, comprising the *M. tuberculosis* structural protein or the immunogenic fragment thereof, or the fusion protein formed by the structural protein or the immunogenic fragment according to claim 3 or 5.

7. A method for preparing the immunogenic complex according to any one of claims 1-5, comprising:
(1) ligating a coding gene of the antigenic component and a coding gene of the particle protein component into expression vectors to construct recombinant expression plasmids and expression host strains, and expressing and purifying the target proteins; and
(2) co-incubating the antigenic component and the particle protein component obtained in step (1) to give the immunogenic complex.

8. An immune composition, comprising the immunogenic complex according to any one of claims 1-5 or the recombinant antigen protein according to claim 6 and a pharmaceutically acceptable carrier, wherein the pharmaceutically acceptable carrier comprises a stabilizer, an excipient, a surfactant, a buffering agent, and a pH regulator; the stabilizer comprises sucrose or arginine, the excipient comprises mannitol, the surfactant comprises Tween 80, the buffering agent comprises disodium hydrogen phosphate dihydrate or sodium dihydrogen phosphate dihydrate, and the pH regulator comprises hydrochloric acid.

9. A *M. tuberculosis* vaccine, comprising the immune composition according to claim 8 and an adjuvant, wherein the adjuvant is selected from at least one of an aluminum salt adjuvant, Freund's complete adjuvant, a propolis adjuvant, an oil-in-water adjuvant, a cytokine, CpGDNA, a genetically engineered toxoid, an immune-stimulating complex, or a liposome.

10. Use of the immunogenic complex according to any one of claims 1-5, the recombinant antigen protein according to claim 6, the immune composition according to claim 8, or the *M. tuberculosis* vaccine according to claim 9 in preparing a medicament for preventing or treating a disease caused by *M. tuberculosis* infection.
